# EUROPEAN PATENT APPLICATION

(11) **EP 0 934 953 A2**
(43) Date of publication of application: **11.08.1999**
(21) Application number: 98122546.9
(22) Date of filing: 01.12.1998
(51) Int. Cl.: C07K 16/00, C12N 15/10, C07K 16/18, G01N 33/53, G01N 33/574

(54) **Complex specific antibodies, method of preparation and uses thereof**

(30) Priority: 03.12.1997 US 67428 P
(71) Applicant: BOEHRINGER MANNHEIM CORPORATION, Indianapolis, IN 46250 (US)
(72) Inventor: Winter, Gregory P., Dr., Cambridge CB2 1TQ (GB); Mahoney, Walter, Dr., California 94510-2523 (US); Sawyer, Jaymie R., Dr., Arizona 85224 (US)
(74) Representative: Schwarz, Ralf, Dr.

(57) **Abstract**

Methods are provided that utilize a naïve antibody library for preparing monoclonal antibodies directed against an antigen complex. In particular, methods are provided for preparing antibodies that show specificity for the PSA-ACT complex (prostate-specific antigen complexed to anti-chymotrypsin) as opposed to the free subunits of the complex. Antibodies prepared by these methods are provided.

SEQUENCE LISTING

(1) GENERAL INFORMATION

(i) APPLICANT: MAHONEY, WALTER
SAWYER, JAYMIE R.
WINTER, GREG

(ii) TITLE OF THE INVENTION: COMPLEX SPECIFIC ANTIBODIES,
METHOD OF PREPARATION AND USES THEREOF

(iii) NUMBER OF SEQUENCES: 25

(iv) CORRESPONDENCE ADDRESS:
(A) ADDRESSEE: MORRISON & FOERSTER
(B) STREET: 755 PAGE MILL ROAD
(C) CITY: PALO ALTO
(D) STATE: CA
(E) COUNTRY: USA
(F) ZIP: 94304-1018

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE: December 3, 1997
(C) CLASSIFICATION:

(vii) PRIOR APPLICATION DATA: None
(A) APPLICATION NUMBER:
(B) FILING DATE:

(viii) ATTORNEY/AGENT INFORMATION:
(A) NAME: TAN, LEE K
(B) REGISTRATION NUMBER: 39,447
(C) REFERENCE/DOCKET NUMBER: 33746-30022.00

(ix) TELECOMMUNICATION INFORMATION:
(A) TELEPHONE: 650-813-5600
(B) TELEFAX: 650-494-0792
(C) TELEX: 706141

(2) INFORMATION FOR SEQ ID NO:1:

(i) SEQUENCE CHARACTERISTICS:
(A) LENGTH: 714 base pairs
(B) TYPE: nucleic acid
(C) STRANDEDNESS: double

(D) TOPOLOGY: linear

(ix) FEATURE:

(A) NAME/KEY: Coding Sequence
(B) LOCATION: 1...714
(D) OTHER INFORMATION:

(xi) SEQUENCE DESCRIPTION: SEQ ID NO:1:

ATG GCC CAG GTG CAG CTG GTG CAG TCT GGG GCT GAG GTG AAG AAG CCT 48
Met Ala Gln Val Gln Leu Val Gln Ser Gly Ala Glu Val Lys Lys Pro
1               5                   10                  15

GGG GCT ACA GTG AAA ATC TCC TGC AAG GTT TCT GGA TAC ACC TTC ACC 96
Gly Ala Thr Val Lys Ile Ser Cys Lys Val Ser Gly Tyr Thr Phe Thr
            20                  25                  30

GAC TAC TAC ATG CAC TGG GTG CAA CAG GCC CCT GGA AAA GGG CTT GAG 144
Asp Tyr Tyr Met His Trp Val Gln Gln Ala Pro Gly Lys Gly Leu Glu
        35                  40                  45

TGG ATG GGA CTT GTT GAT CCT GAA GAT GGT GAA ACA ATA TAC GCA GAG 192
Trp Met Gly Leu Val Asp Pro Glu Asp Gly Glu Thr Ile Tyr Ala Glu
    50                  55                  60

AAG TTC CAG GGC AGA GTC ACC ATA ACC GCG GAC ACG TCT ACA GAC ACA 240
Lys Phe Gln Gly Arg Val Thr Ile Thr Ala Asp Thr Ser Thr Asp Thr
65                  70                  75                  80

GCC TAC ATG GAG CTG AGC AGC CTG AGA TCT GAG GAC ACG GCC GTG TAT 288
Ala Tyr Met Glu Leu Ser Ser Leu Arg Ser Glu Asp Thr Ala Val Tyr
                85                  90                  95

TAC TGT GCA AGA TTG GAT AAT CCG GCT TGG GGC CAA GGT ACC CTG GTC 336
Tyr Cys Ala Arg Leu Asp Asn Pro Ala Trp Gly Gln Gly Thr Leu Val
            100                 105                 110

ACC GTC TCG AGT GGT GGA GGC GGT TCA GGC GGA GGT GGC TCT GGC GGT 384
Thr Val Ser Ser Gly Gly Gly Gly Ser Gly Gly Gly Gly Ser Gly Gly
        115                 120                 125

AGT GCA CTT GAC ATC CAG TTG ACC CAG TCT CCA TCC TCC CTG TCT GCA 432
Ser Ala Leu Asp Ile Gln Leu Thr Gln Ser Pro Ser Ser Leu Ser Ala
    130                 135                 140

TCT GTA GGA GAC AGA GTC ACC ATC ACT TGC CAG GCG AGT CAG GAC ATT 480
Ser Val Gly Asp Arg Val Thr Ile Thr Cys Gln Ala Ser Gln Asp Ile
145                 150                 155                 160

AGC AAC TAT TTA AAT TGG TAT CAG CAG AAA CCA GGG AAA GCC CCT AAG 528
Ser Asn Tyr Leu Asn Trp Tyr Gln Gln Lys Pro Gly Lys Ala Pro Lys
                165                 170                 175

CTC CTG ATC TAC GAT GCA TCC AAT TTG GAA ACA GGG GTC CCA TCA AGG 576
Leu Leu Ile Tyr Asp Ala Ser Asn Leu Glu Thr Gly Val Pro Ser Arg
            180                 185                 190

TTC AGT GGA AGT GGA TCT GGG ACA GAT TTT ACT TTC ACC ATC AGC AGC 624
Phe Ser Gly Ser Gly Ser Gly Thr Asp Phe Thr Phe Thr Ile Ser Ser
        195                 200                 205

CTG CAG TCT GAA GAT ATT GCA ACA TAT TAC TGT CAA CAG TAT GAT AAT 672
Leu Gln Ser Glu Asp Ile Ala Thr Tyr Tyr Cys Gln Gln Tyr Asp Asn
    210                 215                 220

CTC CCC ACG TTC GGC CAA GGG ACC AAG CTG GAA ATC AAA CGT 714
Leu Pro Thr Phe Gly Gln Gly Thr Lys Leu Glu Ile Lys Arg
225                 230                 235

(2) INFORMATION FOR SEQ ID NO:2:

(i) SEQUENCE CHARACTERISTICS:
(A) LENGTH: 238 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:2:

Met Ala Gln Val Gln Leu Val Gln Ser Gly Ala Glu Val Lys Lys Pro
1               5                   10                  15
Gly Ala Thr Val Lys Ile Ser Cys Lys Val Ser Gly Tyr Thr Phe Thr
            20                  25                  30
Asp Tyr Tyr Met His Trp Val Gln Gln Ala Pro Gly Lys Gly Leu Glu
        35                  40                  45
Trp Met Gly Leu Val Asp Pro Glu Asp Gly Glu Thr Ile Tyr Ala Glu
    50                  55                  60
Lys Phe Gln Gly Arg Val Thr Ile Thr Ala Asp Thr Ser Thr Asp Thr
65                  70                  75                  80
Ala Tyr Met Glu Leu Ser Ser Leu Arg Ser Glu Asp Thr Ala Val Tyr
                85                  90                  95
Tyr Cys Ala Arg Leu Asp Asn Pro Ala Trp Gly Gln Gly Thr Leu Val
            100                 105                 110
Thr Val Ser Ser Gly Gly Gly Gly Ser Gly Gly Gly Gly Ser Gly Gly
        115                 120                 125
Ser Ala Leu Asp Ile Gln Leu Thr Gln Ser Pro Ser Ser Leu Ser Ala
    130                 135                 140

Ser Val Gly Asp Arg Val Thr Ile Thr Cys Gln Ala Ser Gln Asp Ile
145                 150                 155                 160
Ser Asn Tyr Leu Asn Trp Tyr Gln Gln Lys Pro Gly Lys Ala Pro Lys
                165                 170                 175
Leu Leu Ile Tyr Asp Ala Ser Asn Leu Glu Thr Gly Val Pro Ser Arg
            180                 185                 190
Phe Ser Gly Ser Gly Ser Gly Thr Asp Phe Thr Phe Thr Ile Ser Ser
        195                 200                 205
Leu Gln Ser Glu Asp Ile Ala Thr Tyr Tyr Cys Gln Gln Tyr Asp Asn
    210                 215                 220
Leu Pro Thr Phe Gly Gln Gly Thr Lys Leu Glu Ile Lys Arg
225                 230                 235

(2) INFORMATION FOR SEQ ID NO:3:

(i) SEQUENCE CHARACTERISTICS:
(A) LENGTH: 714 base pairs
(B) TYPE: nucleic acid
(C) STRANDEDNESS: double
(D) TOPOLOGY: linear

(xi) SEQUENCE DESCRIPTION: SEQ ID NO:3:

ACGTTTGATT TCCAGCTTGG TCCCTTGGCC GAACGTGGGG AGATTATCAT ACTGTTGACA 60
GTAATATGTT GCAATATCTT CAGACTGCAG GCTGCTGATG GTGAAAGTAA AATCTGTCCC 120
AGATCCACTT CCACTGAACC TTGATGGGAC CCCTGTTTCC AAATTGGATG CATCGTAGAT 180
CAGGAGCTTA GGGGCTTTCC CTGGTTTCTG CTGATACCAA TTTAAATAGT TGCTAATGTC 240
CTGACTCGCC TGGCAAGTGA TGGTGACTCT GTCTCCTACA GATGCAGACA GGGAGGATGG 300
AGACTGGGTC AACTGGATGT CAAGTGCACT ACCGCCAGAG CCACCTCCGC CTGAACCGCC 360
TCCACCACTC GAGACGGTGA CCAGGGTACC TTGGCCCCAA GCCGGATTAT CCAATCTTGC 420
ACAGTAATAC ACGGCCGTGT CCTCAGATCT CAGGCTGCTC AGCTCCATGT AGGCTGTGTC 480
TGTAGACGTG TCCGCGGTTA TGGTGACTCT GCCCTGGAAC TTCTCTGCGT ATATTGTTTC 540
ACCATCTTCA GGATCAACAA GTCCCATCCA CTCAAGCCCT TTTCCAGGGG CCTGTTGCAC 600
CCAGTGCATG TAGTAGTCGG TGAAGGTGTA TCCAGAAACC TTGCAGGAGA TTTTCACTGT 660
AGCCCCAGGC TTCTTCACCT CAGCCCCAGA CTGCACCAGC TGCACCTGGG CCAT 714

(2) INFORMATION FOR SEQ ID NO:4:

(i) SEQUENCE CHARACTERISTICS:
(A) LENGTH: 726 base pairs
(B) TYPE: nucleic acid
(C) STRANDEDNESS: double
(D) TOPOLOGY: linear

(ix) FEATURE:

(A) NAME/KEY: Coding Sequence
(B) LOCATION: 1...726
(D) OTHER INFORMATION:

(xi) SEQUENCE DESCRIPTION: SEQ ID NO:4:

ATG GCC GAG GTG CAG CTG GTG GAG TCT GGG GGA GGC TTG GTA CAG CCT 48
Met Ala Glu Val Gln Leu Val Glu Ser Gly Gly Gly Leu Val Gln Pro
1               5                   10                  15

GGG GGG TCC CTG AGA CTC TCC TGT GCA GCC TCT GGA TTC ACC TTT AGC 96
Gly Gly Ser Leu Arg Leu Ser Cys Ala Ala Ser Gly Phe Thr Phe Ser
            20                  25                  30

AGC TAT GCC ATG AGC TGG GTC CGC CAG GCT CCA GGG AAG GGG CTG GAG 144
Ser Tyr Ala Met Ser Trp Val Arg Gln Ala Pro Gly Lys Gly Leu Glu
        35                  40                  45

TGG GTC TCA GCT ATT AGT GGT AGT GGT GGT AGC ACA TAC TAC GCA GAC 192
Trp Val Ser Ala Ile Ser Gly Ser Gly Gly Ser Thr Tyr Tyr Ala Asp
    50                  55                  60

TCC GTG AAG GGC CGG TTC ACC ATC TCC AGA GAC AAC GCC AAG AAG TCC 240
Ser Val Lys Gly Arg Phe Thr Ile Ser Arg Asp Asn Ala Lys Lys Ser
65                  70                  75                  80

TTG TAT CTT CAA ATG AAC AGC CTG ATA GCT GAG GAC ACG GCC GTG TAT 288
Leu Tyr Leu Gln Met Asn Ser Leu Ile Ala Glu Asp Thr Ala Val Tyr
                85                  90                  95

TAC TGT GCA AGA TCT TCG GGG CAT ATG TGG GGC CAA GGT ACC CTG GTC 336
Tyr Cys Ala Arg Ser Ser Gly His Met Trp Gly Gln Gly Thr Leu Val
            100                 105                 110

ACC GTC TCG AGT GGT GGA GGC GGT TCA GGC GGA GGT GGC TCT GGC GGT 384
Thr Val Ser Ser Gly Gly Gly Gly Ser Gly Gly Gly Gly Ser Gly Gly
        115                 120                 125

AGT GCA CTT CAG TCT GTG CTG ACT CAG CCA CCC TCA GCG TCT GGG ACC 432
Ser Ala Leu Gln Ser Val Leu Thr Gln Pro Pro Ser Ala Ser Gly Thr
    130                 135                 140

CCC GGG CAG AGG GTC ACC ATC TCT TGT TCT GGA AGC AGC TCC AAC ATC 480
Pro Gly Gln Arg Val Thr Ile Ser Cys Ser Gly Ser Ser Ser Asn Ile
145                 150                 155                 160

GGA AGT AAT TAT GTA TAC TGG TAC CAG CAG CTC CCA GGA ACG GCC CCC 528
Gly Ser Asn Tyr Val Tyr Trp Tyr Gln Gln Leu Pro Gly Thr Ala Pro
                165                 170                 175

AAA CTC CTC ATC TAT AGG AAT AAT CAG CGG CCC TCA GGG GTC CCT GAC 576
Lys Leu Leu Ile Tyr Arg Asn Asn Gln Arg Pro Ser Gly Val Pro Asp
            180                 185                 190

CGA TTC TCT GGC TCC AAG TCT GGC ACC TCA GCC TCC CTG GCC ATC AGT 624
Arg Phe Ser Gly Ser Lys Ser Gly Thr Ser Ala Ser Leu Ala Ile Ser
        195                 200                 205

GGG CTC CGG TCC GAG GAT GAG GCT GAT TAT TAC TGT GCA GCA TGG GAT 672
Gly Leu Arg Ser Glu Asp Glu Ala Asp Tyr Tyr Cys Ala Ala Trp Asp
    210                 215                 220

GAC AGC CTG TCT GCG CTT GTA TTC GGC GGA GGG ACC AAG CTG ACC GTC 720
Asp Ser Leu Ser Ala Leu Val Phe Gly Gly Gly Thr Lys Leu Thr Val
225                 230                 235                 240

CTA GGT 726
Leu Gly

(2) INFORMATION FOR SEQ ID NO:5:

(i) SEQUENCE CHARACTERISTICS:
(A) LENGTH: 242 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:5:

Met Ala Glu Val Gln Leu Val Glu Ser Gly Gly Gly Leu Val Gln Pro
1               5                   10                  15
Gly Gly Ser Leu Arg Leu Ser Cys Ala Ala Ser Gly Phe Thr Phe Ser
            20                  25                  30
Ser Tyr Ala Met Ser Trp Val Arg Gln Ala Pro Gly Lys Gly Leu Glu
        35                  40                  45
Trp Val Ser Ala Ile Ser Gly Ser Gly Gly Ser Thr Tyr Tyr Ala Asp
    50                  55                  60
Ser Val Lys Gly Arg Phe Thr Ile Ser Arg Asp Asn Ala Lys Lys Ser
65                  70                  75                  80
Leu Tyr Leu Gln Met Asn Ser Leu Ile Ala Glu Asp Thr Ala Val Tyr
                85                  90                  95
Tyr Cys Ala Arg Ser Ser Gly His Met Trp Gly Gln Gly Thr Leu Val
            100                 105                 110
Thr Val Ser Ser Gly Gly Gly Gly Ser Gly Gly Gly Gly Ser Gly Gly
        115                 120                 125
Ser Ala Leu Gln Ser Val Leu Thr Gln Pro Pro Ser Ala Ser Gly Thr
    130                 135                 140
Pro Gly Gln Arg Val Thr Ile Ser Cys Ser Gly Ser Ser Ser Asn Ile
145                 150                 155                 160
Gly Ser Asn Tyr Val Tyr Trp Tyr Gln Gln Leu Pro Gly Thr Ala Pro
                165                 170                 175
Lys Leu Leu Ile Tyr Arg Asn Asn Gln Arg Pro Ser Gly Val Pro Asp
            180                 185                 190

Arg Phe Ser Gly Ser Lys Ser Gly Thr Ser Ala Ser Leu Ala Ile Ser
      195                 200                 205
Gly Leu Arg Ser Glu Asp Glu Ala Asp Tyr Tyr Cys Ala Ala Trp Asp
    210                 215                 220
Asp Ser Leu Ser Ala Leu Val Phe Gly Gly Gly Thr Lys Leu Thr Val
225                 230                 235                 240
Leu Gly

(2) INFORMATION FOR SEQ ID NO:6:

(i) SEQUENCE CHARACTERISTICS:
(A) LENGTH: 726 base pairs
(B) TYPE: nucleic acid
(C) STRANDEDNESS: double
(D) TOPOLOGY: linear

(xi) SEQUENCE DESCRIPTION: SEQ ID NO:6:

ACCTAGGACG GTCAGCTTGG TCCCTCCGCC GAATACAAGC GCAGACAGGC TGTCATCCCA 60
TGCTGCACAG TAATAATCAG CCTCATCCTC GGACCGGAGC CCACTGATGG CCAGGGAGGC 120
TGAGGTGCCA GACTTGGAGC CAGAGAATCG GTCAGGGACC CCTGAGGGCC GCTGATTATT 180
CCTATAGATG AGGAGTTTGG GGGCCGTTCC TGGGAGCTGC TGGTACCAGT ATACATAATT 240
ACTTCCGATG TTGGAGCTGC TTCCAGAACA AGAGATGGTG ACCCTCTGCC CGGGGGTCCC 300
AGACGCTGAG GGTGGCTGAG TCAGCACAGA CTGAAGTGCA CTACCGCCAG AGCCACCTCC 360
GCCTGAACCG CCTCCACCAC TCGAGACGGT GACCAGGGTA CCTTGGCCCC ACATATGCCC 420
CGAAGATCTT GCACAGTAAT ACACGGCCGT GTCCTCAGCT ATCAGGCTGT TCATTTGAAG 480
ATACAAGGAC TTCTTGGCGT TGTCTCTGGA GATGGTGAAC CGGCCCTTCA CGGAGTCTGC 540
GTAGTATGTG CTACCACCAC TACCACTAAT AGCTGAGACC CACTCCAGCC CCTTCCCTGG 600
AGCCTGGCGG ACCCAGCTCA TGGCATAGCT GCTAAAGGTG AATCCAGAGG CTGCACAGGA 660
GAGTCTCAGG GACCCCCCAG GCTGTACCAA GCCTCCCCCA GACTCCACCA GCTGCACCTC 720
GGCCAT 726

(2) INFORMATION FOR SEQ ID NO:7:

(i) SEQUENCE CHARACTERISTICS:
(A) LENGTH: 723 base pairs
(B) TYPE: nucleic acid
(C) STRANDEDNESS: double
(D) TOPOLOGY: linear

(ix) FEATURE:

(A) NAME/KEY: Coding Sequence
(B) LOCATION: 1...723
(D) OTHER INFORMATION:

(A) NAME/KEY: Modified Base
(B) LOCATION: 304...0
(D) OTHER INFORMATION: N=T

(xi) SEQUENCE DESCRIPTION: SEQ ID NO:7:

ATG GCC CAG GTG CAG CTG GTG CAG TCT GGG GCT GAG GTG AAG AAG CCT 48
Met Ala Gln Val Gln Leu Val Gln Ser Gly Ala Glu Val Lys Lys Pro
1               5                   10                  15

GGG GCC TCA GTG AAG GTT TCC TGC AAG GCA TCT GGA TAC ACC TTC ACC 96
Gly Ala Ser Val Lys Val Ser Cys Lys Ala Ser Gly Tyr Thr Phe Thr
            20                  25                  30

GAC TAC TAC ATG CAC TGG GTG CAA CAG GCC CCT GGA AAA GGG CTT GAG 144
Asp Tyr Tyr Met His Trp Val Gln Gln Ala Pro Gly Lys Gly Leu Glu
        35                  40                  45

TGG ATG GGA CTT GTT GAT CCT GAA GAT GGT GAA ACA ATA TAC GCA GAG 192
Trp Met Gly Leu Val Asp Pro Glu Asp Gly Glu Thr Ile Tyr Ala Glu
    50                  55                  60

AAG TTC CAG GGC AGA GTC ACC ATA ACC GCG GAC ACG TCT ACA GAC ACA 240
Lys Phe Gln Gly Arg Val Thr Ile Thr Ala Asp Thr Ser Thr Asp Thr
65                  70                  75                  80

GCC TAC ATG GAG CTG AGC AGC CTG AGA TCT GAG GAC ACG GCC GTG TAT 288
Ala Tyr Met Glu Leu Ser Ser Leu Arg Ser Glu Asp Thr Ala Val Tyr
                85                  90                  95

TAC TGT GCA AGA GAG NAG GCT CCT GCG TTT GAC TAT TGG GGC CAA GGT 336
Tyr Cys Ala Arg Glu Xaa Ala Pro Ala Phe Asp Tyr Trp Gly Gln Gly
            100                 105                 110

ACC CTG GTC ACC GTC TCG AGT GGT GGA GGC GGT TCA GGC GGA GGT GGC 384
Thr Leu Val Thr Val Ser Ser Gly Gly Gly Gly Ser Gly Gly Gly Gly
        115                 120                 125

TCT GGC GGT AGT GCA CTT GAC ATC CAG ATG ACC CAG TCT CCA TCC TCC 432
Ser Gly Gly Ser Ala Leu Asp Ile Gln Met Thr Gln Ser Pro Ser Ser
    130                 135                 140

CTG TCT GCA TCT GTA GGA GAC AGA GTC ACC ATC ACT TGC CAG GCG AGT 480
Leu Ser Ala Ser Val Gly Asp Arg Val Thr Ile Thr Cys Gln Ala Ser
145                 150                 155                 160

CAG GAC ATT AGC AAC TAT TTA AAT TGG TAT CAG CAG AAA CCA GGG AAA 528
Gln Asp Ile Ser Asn Tyr Leu Asn Trp Tyr Gln Gln Lys Pro Gly Lys
                165                 170                 175

GCC CCT AAG CTC CTG ATC TAC GAT GCA TCC AAT TTG GAG ACA GGG GTC 576
Ala Pro Lys Leu Leu Ile Tyr Asp Ala Ser Asn Leu Glu Thr Gly Val
            180                 185                 190

CCA TCA AGG TTC AGT GGA AGT GGA TCT GGG ACA GAT TTT ACT TTC ACC 624
Pro Ser Arg Phe Ser Gly Ser Gly Ser Gly Thr Asp Phe Thr Phe Thr
        195                 200                 205

ATC AGC AGC CTG CAG CCT GAA GAT ATT GCA ACA TAT TAC TGT CAA CAG 672
Ile Ser Ser Leu Gln Pro Glu Asp Ile Ala Thr Tyr Tyr Cys Gln Gln
    210                 215                 220

TAT GAT AAT CTC CCC ACG TTC GGC CAA GGG ACC AAG GTG GAA ATC AAA 720
Tyr Asp Asn Leu Pro Thr Phe Gly Gln Gly Thr Lys Val Glu Ile Lys
225                 230                 235                 240

CGT 723
Arg

(2) INFORMATION FOR SEQ ID NO:8:

(i) SEQUENCE CHARACTERISTICS:
(A) LENGTH: 241 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:8:

Met Ala Gln Val Gln Leu Val Gln Ser Gly Ala Glu Val Lys Lys Pro
1               5                   10                  15
Gly Ala Ser Val Lys Val Ser Cys Lys Ala Ser Gly Tyr Thr Phe Thr
            20                  25                  30
Asp Tyr Tyr Met His Trp Val Gln Gln Ala Pro Gly Lys Gly Leu Glu
        35                  40                  45
Trp Met Gly Leu Val Asp Pro Glu Asp Gly Glu Thr Ile Tyr Ala Glu
    50                  55                  60
Lys Phe Gln Gly Arg Val Thr Ile Thr Ala Asp Thr Ser Thr Asp Thr
65                  70                  75                  80
Ala Tyr Met Glu Leu Ser Ser Leu Arg Ser Glu Asp Thr Ala Val Tyr
                85                  90                  95
Tyr Cys Ala Arg Glu Xaa Ala Pro Ala Phe Asp Tyr Trp Gly Gln Gly
            100                 105                 110
Thr Leu Val Thr Val Ser Ser Gly Gly Gly Gly Ser Gly Gly Gly Gly
        115                 120                 125
Ser Gly Gly Ser Ala Leu Asp Ile Gln Met Thr Gln Ser Pro Ser Ser
    130                 135                 140
Leu Ser Ala Ser Val Gly Asp Arg Val Thr Ile Thr Cys Gln Ala Ser
145                 150                 155                 160
Gln Asp Ile Ser Asn Tyr Leu Asn Trp Tyr Gln Gln Lys Pro Gly Lys
                165                 170                 175
Ala Pro Lys Leu Leu Ile Tyr Asp Ala Ser Asn Leu Glu Thr Gly Val
            180                 185                 190

Pro Ser Arg Phe Ser Gly Ser Gly Ser Gly Thr Asp Phe Thr Phe Thr
        195                 200                 205
Ile Ser Ser Leu Gln Pro Glu Asp Ile Ala Thr Tyr Tyr Cys Gln Gln
    210                 215                 220
Tyr Asp Asn Leu Pro Thr Phe Gly Gln Gly Thr Lys Val Glu Ile Lys
225                 230                 235                 240
Arg

(2) INFORMATION FOR SEQ ID NO:9:

(i) SEQUENCE CHARACTERISTICS:
(A) LENGTH: 723 base pairs
(B) TYPE: nucleic acid
(C) STRANDEDNESS: double
(D) TOPOLOGY: linear

(xi) SEQUENCE DESCRIPTION: SEQ ID NO:9:

ACGTTTGATT TCCACCTTGG TCCCTTGGCC GAACGTGGGG AGATTATCAT ACTGTTGACA 60
GTAATATGTT GCAATATCTT CAGGCTGCAG GCTGCTGATG GTGAAAGTAA AATCTGTCCC 120
AGATCCACTT CCACTGAACC TTGATGGGAC CCCTGTCTCC AAATTGGATG CATCGTAGAT 180
CAGGAGCTTA GGGGCTTTCC CTGGTTTCTG CTGATACCAA TTTAAATAGT TGCTAATGTC 240
CTGACTCGCC TGGCAAGTGA TGGTGACTCT GTCTCCTACA GATGCAGACA GGGAGGATGG 300
AGACTGGGTC ATCTGGATGT CAAGTGCACT ACCGCCAGAG CCACCTCCGC CTGAACCGCC 360
TCCACCACTC GAGACGGTGA CCAGGGTACC TTGGCCCCAA TAGTCAAACG CAGGAGCCTA 420
CTCTCTTGCA CAGTAATACA CGGCCGTGTC CTCAGATCTC AGGCTGCTCA GCTCCATGTA 480
GGCTGTGTCT GTAGACGTGT CCGCGGTTAT GGTGACTCTG CCCTGGAACT TCTCTGCGTA 540
TATTGTTTCA CCATCTTCAG GATCAACAAG TCCCATCCAC TCAAGCCCTT TTCCAGGGGC 600
CTGTTGCACC CAGTGCATGT AGTAGTCGGT GAAGGTGTAT CCAGATGCCT TGCAGGAAAC 660
CTTCACTGAG GCCCCAGGCT TCTTCACCTC AGCCCCAGAC TGCACCAGCT GCACCTGGGC 720
CAT 723

(2) INFORMATION FOR SEQ ID NO:10:

(i) SEQUENCE CHARACTERISTICS:
(A) LENGTH: 732 base pairs
(B) TYPE: nucleic acid
(C) STRANDEDNESS: double
(D) TOPOLOGY: linear

(ix) FEATURE:

(A) NAME/KEY: Coding Sequence
(B) LOCATION: 1...732
(D) OTHER INFORMATION:

(xi) SEQUENCE DESCRIPTION: SEQ ID NO:10:

ATG GCC CAG GTG CAG CTG CAG GAG TCG GGC GCA GGA CTG TTG AAG CCT 48
Met Ala Gln Val Gln Leu Gln Glu Ser Gly Ala Gly Leu Leu Lys Pro
1               5                   10                  15

TCG GAG ACC CTG TCC CTC ACC TGC GCT GTC TAT GGT GGG TCC TTC AGT 96
Ser Glu Thr Leu Ser Leu Thr Cys Ala Val Tyr Gly Gly Ser Phe Ser
            20                  25                  30

GGT TAC TAC TGG AGC TGG ATC CGC CAG CCC CCA GGG AAG GGG CTG GAG 144
Gly Tyr Tyr Trp Ser Trp Ile Arg Gln Pro Pro Gly Lys Gly Leu Glu
        35                  40                  45

TGG ATT GGG GAA ATC AAT CAT AGT GGA AGC ACC AAC TAC AAC CCG TCC 192
Trp Ile Gly Glu Ile Asn His Ser Gly Ser Thr Asn Tyr Asn Pro Ser
    50                  55                  60

CTC AAG AGT CGA GTC ACC ATA TCA GTA GAC ACG TCC AAG AAC CAG TTC 240
Leu Lys Ser Arg Val Thr Ile Ser Val Asp Thr Ser Lys Asn Gln Phe
65                  70                  75                  80

TCC CTG AAG CTG AGC TCT GTG ACC GCC GCG GAC ACG GCC GTG TAT TAC 288
Ser Leu Lys Leu Ser Ser Val Thr Ala Ala Asp Thr Ala Val Tyr Tyr
                85                  90                  95

TGT GCA AGA ATG TGG TCT CTG AAG TTT GAC TAT TGG GGC CAA GGT ACC 336
Cys Ala Arg Met Trp Ser Leu Lys Phe Asp Tyr Trp Gly Gln Gly Thr
            100                 105                 110

CTG GTC ACC GTC TCG AGT GGT GGA GGC GGT TCA GGC GGA GGT GGC TCT 384
Leu Val Thr Val Ser Ser Gly Gly Gly Gly Ser Gly Gly Gly Gly Ser
        115                 120                 125

GGC GGT AGT GCA CTT CAG TCT GTG CTG ACT CAG CCA CCC TCA GCG TCT 432
Gly Gly Ser Ala Leu Gln Ser Val Leu Thr Gln Pro Pro Ser Ala Ser
    130                 135                 140

GGG ACC CCC GGG CAG AGG GTC ACC ATC TCT TGT TCT GGA AGC AGC TCC 480
Gly Thr Pro Gly Gln Arg Val Thr Ile Ser Cys Ser Gly Ser Ser Ser
145                 150                 155                 160

AAC ATC GGA AGT AAT TAT GTA TAC TGG TAC CAG CAG CTC CCA GGA ACG 528
Asn Ile Gly Ser Asn Tyr Val Tyr Trp Tyr Gln Gln Leu Pro Gly Thr
                165                 170                 175

GCC CCC AAA CTC CTC ATC TAT AGG AAT AAT CAG CGG CCC TCA GGG GTC 576
Ala Pro Lys Leu Leu Ile Tyr Arg Asn Asn Gln Arg Pro Ser Gly Val
            180                 185                 190

CCT GAC CGA TTC TCT GGC TCC AAG TST GGC ACC TCA GCC TCC CTG GCC 624
Pro Asp Arg Phe Ser Gly Ser Lys Xaa Gly Thr Ser Ala Ser Leu Ala
        195                 200                 205

ATC AGT GGG CTC CGG TCC GAG GAT GAG GCT GAT TAT TAC TGT GCT GCA 672
Ile Ser Gly Leu Arg Ser Glu Asp Glu Ala Asp Tyr Tyr Cys Ala Ala
    210                 215                 220

TGG GAT GAC AGC CTG CGT GGT ATT GTA TTC GGC GGA GGG ACC AAG CTG 720
Trp Asp Asp Ser Leu Arg Gly Ile Val Phe Gly Gly Gly Thr Lys Leu
225                 230                 235                 240

ACC GTC CTA GGT 732
Thr Val Leu Gly

(2) INFORMATION FOR SEQ ID NO:11:

(i) SEQUENCE CHARACTERISTICS:
(A) LENGTH: 244 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:11:

Met Ala Gln Val Gln Leu Gln Glu Ser Gly Ala Gly Leu Leu Lys Pro
1               5                   10                  15
Ser Glu Thr Leu Ser Leu Thr Cys Ala Val Tyr Gly Gly Ser Phe Ser
            20                  25                  30
Gly Tyr Tyr Trp Ser Trp Ile Arg Gln Pro Pro Gly Lys Gly Leu Glu
        35                  40                  45
Trp Ile Gly Glu Ile Asn His Ser Gly Ser Thr Asn Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Met Trp Ser Leu Lys Phe Asp Tyr Trp Gly Gln Gly Thr
            100                 105                 110
Leu Val Thr Val Ser Ser Gly Gly Gly Gly Ser Gly Gly Gly Gly Ser
        115                 120                 125
Gly Gly Ser Ala Leu Gln Ser Val Leu Thr Gln Pro Pro Ser Ala Ser
    130                 135                 140
Gly Thr Pro Gly Gln Arg Val Thr Ile Ser Cys Ser Gly Ser Ser Ser
145                 150                 155                 160
Asn Ile Gly Ser Asn Tyr Val Tyr Trp Tyr Gln Gln Leu Pro Gly Thr
                165                 170                 175
Ala Pro Lys Leu Leu Ile Tyr Arg Asn Asn Gln Arg Pro Ser Gly Val
            180                 185                 190
Pro Asp Arg Phe Ser Gly Ser Lys Xaa Gly Thr Ser Ala Ser Leu Ala
        195                 200                 205
Ile Ser Gly Leu Arg Ser Glu Asp Glu Ala Asp Tyr Tyr Cys Ala Ala
    210                 215                 220
Trp Asp Asp Ser Leu Arg Gly Ile Val Phe Gly Gly Gly Thr Lys Leu
225                 230                 235                 240
Thr Val Leu Gly

(2) INFORMATION FOR SEQ ID NO:12:

(i) SEQUENCE CHARACTERISTICS:
(A) LENGTH: 732 base pairs
(B) TYPE: nucleic acid
(C) STRANDEDNESS: double
(D) TOPOLOGY: linear

(xi) SEQUENCE DESCRIPTION: SEQ ID NO:12:

ACCTAGGACG GTCAGCTTGG TCCCTCCGCC GAATACAATA CCACGCAGGC TGTCATCCCA 60
TGCAGCACAG TAATAATCAG CCTCATCCTC GGACCGGAGC CCACTGATGG CCAGGGAGGC 120
TGAGGTGCCA SACTTGGAGC CAGAGAATCG GTCAGGGACC CCTGAGGGCC GCTGATTATT 180
CCTATAGATG AGGAGTTTGG GGGCCGTTCC TGGGAGCTGC TGGTACCAGT ATACATAATT 240
ACTTCCGATG TTGGAGCTGC TTCCAGAACA AGAGATGGTG ACCCTCTGCC CGGGGGTCCC 300
AGACGCTGAG GGTGGCTGAG TCAGCACAGA CTGAAGTGCA CTACCGCCAG AGCCACCTCC 360
GCCTGAACCG CCTCCACCAC TCGAGACGGT GACCAGGGTA CCTTGGCCCC AATAGTCAAA 420
CTTCAGAGAC CACATTCTTG CACAGTAATA CACGGCCGTG TCCGCGGCGG TCACAGAGCT 480
CAGCTTCAGG GAGAACTGGT TCTTGGACGT GTCTACTGAT ATGGTGACTC GACTCTTGAG 540
GGACGGGTTG TAGTTGGTGC TTCCACTATG ATTGATTTCC CCAATCCACT CCAGCCCCTT 600
CCCTGGGGGC TGGCGGATCC AGCTCCAGTA GTAACCACTG AAGGACCCAC CATAGACAGC 660
GCAGGTGAGG GACAGGGTCT CCGAAGGCTT CAACAGTCCT GCGCCCGACT CCTGCAGCTG 720
CACCTGGGCC AT 732

(2) INFORMATION FOR SEQ ID NO:13:

(i) SEQUENCE CHARACTERISTICS:
(A) LENGTH: 726 base pairs
(B) TYPE: nucleic acid
(C) STRANDEDNESS: double
(D) TOPOLOGY: linear

(ix) FEATURE:

(A) NAME/KEY: Coding Sequence
(B) LOCATION: 1...726
(D) OTHER INFORMATION:

(xi) SEQUENCE DESCRIPTION: SEQ ID NO:13:

ATG GCC CAG GTG CAG CTG GTG CAG TCT GGG GCT GAG GTG AAG AAG CCT 48
Met Ala Gln Val Gln Leu Val Gln Ser Gly Ala Glu Val Lys Lys Pro
1               5                   10                  15

GGG GCC TCA GTG AAG GTC TCC TGC AAG GCT TCT GGT TAC ACC TTT ACC 96
Gly Ala Ser Val Lys Val Ser Cys Lys Ala Ser Gly Tyr Thr Phe Thr
            20                  25                  30

AGC TAT GGT ATC AGC TGG GTG CGA CAG GCC CCT GGA CAA GGG CTT GAG 144
Ser Tyr Gly Ile Ser Trp Val Arg Gln Ala Pro Gly Gln Gly Leu Glu
        35                  40                  45

TGG ATG GGA TGG ATC AGC GCT TAC AAT GGT AAC ACA AAA TAT TCA CAG 192
Trp Met Gly Trp Ile Ser Ala Tyr Asn Gly Asn Thr Lys Tyr Ser Gln
    50                  55                  60

AAG TTC CAG GGC AGA GTC ACC ATT ACC AGG GAC ACA TCC GCG AGC ACA 240
Lys Phe Gln Gly Arg Val Thr Ile Thr Arg Asp Thr Ser Ala Ser Thr
65                  70                  75                  80

GCC TAC ATG GAG CTG AGC AGC CTG AGA TCT GAA GAC ACG GCC GTG TAT 288
Ala Tyr Met Glu Leu Ser Ser Leu Arg Ser Glu Asp Thr Ala Val Tyr
                85                  90                  95

TAC TGT GCA AGA GGG AST CGG TTT TGG GGC CAA GGT ACC CTG GTC ACC 336
Tyr Cys Ala Arg Gly Xaa Arg Phe Trp Gly Gln Gly Thr Leu Val Thr
            100                 105                 110

GTC TCN AGT GGT GGA GGC GGT TCA GGC GGA GGT GGC TCT GGC GGT AGT 384
Val Xaa Ser Gly Gly Gly Gly Ser Gly Gly Gly Gly Ser Gly Gly Ser
        115                 120                 125

GCA CTT GAT ATT GTG ATG ACC CAG ACT CCA CTC TCT CTG TCC GTC ACC 432
Ala Leu Asp Ile Val Met Thr Gln Thr Pro Leu Ser Leu Ser Val Thr
    130                 135                 140

CCT GGA CAG CCG GCC TCC ATC TCC TGC AAG TST AGT CAG AAC CTC CTG 480
Pro Gly Gln Pro Ala Ser Ile Ser Cys Lys Xaa Ser Gln Asn Leu Leu
145                 150                 155                 160

CAT AGT GAT GGA AAG ACC TAT TTG TAT TGG TAC CTG CAG AAG CCA GGC 528
His Ser Asp Gly Lys Thr Tyr Leu Tyr Trp Tyr Leu Gln Lys Pro Gly
                165                 170                 175

CAG CCT CCA CAG CTC CTG ATC TAT GAA GTT TCC AAC CGG TTC TCT GGA 576
Gln Pro Pro Gln Leu Leu Ile Tyr Glu Val Ser Asn Arg Phe Ser Gly
            180                 185                 190

GTG CCA GAT AGG TTC AGT GGC AGC GGG TCA GGG ACA GAT TTC ACA CTG 624
Val Pro Asp Arg Phe Ser Gly Ser Gly Ser Gly Thr Asp Phe Thr Leu
        195                 200                 205

AAA ATC AGC CGG GTG GAG GCT GAG GAT GTT GGG GTT TAT TAC TGC ATG 672
Lys Ile Ser Arg Val Glu Ala Glu Asp Val Gly Val Tyr Tyr Cys Met
    210                 215                 220

CAA AGT ATA CAG CTT CAC TCG TTC GGC CAA GGG ACC AAG GTG GAA ATC 720
Gln Ser Ile Gln Leu His Ser Phe Gly Gln Gly Thr Lys Val Glu Ile
225                 230                 235                 240

AAA CGT 726
Lys Arg

(2) INFORMATION FOR SEQ ID NO:14:

(i) SEQUENCE CHARACTERISTICS:
(A) LENGTH: 242 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:14:

Met Ala Gln Val Gln Leu Val Gln Ser Gly Ala Glu Val Lys Lys Pro
1               5                   10                  15
Gly Ala Ser Val Lys Val Ser Cys Lys Ala Ser Gly Tyr Thr Phe Thr
            20                  25                  30
Ser Tyr Gly Ile Ser Trp Val Arg Gln Ala Pro Gly Gln Gly Leu Glu
        35                  40                  45
Trp Met Gly Trp Ile Ser Ala Tyr Asn Gly Asn Thr Lys Tyr Ser Gln
    50                  55                  60
Lys Phe Gln Gly Arg Val Thr Ile Thr Arg Asp Thr Ser Ala Ser Thr
65                  70                  75                  80
Ala Tyr Met Glu Leu Ser Ser Leu Arg Ser Glu Asp Thr Ala Val Tyr
                85                  90                  95
Tyr Cys Ala Arg Gly Xaa Arg Phe Trp Gly Gln Gly Thr Leu Val Thr
            100                 105                 110
Val Xaa Ser Gly Gly Gly Gly Ser Gly Gly Gly Gly Ser Gly Gly Ser
        115                 120                 125
Ala Leu Asp Ile Val Met Thr Gln Thr Pro Leu Ser Leu Ser Val Thr
    130                 135                 140
Pro Gly Gln Pro Ala Ser Ile Ser Cys Lys Xaa Ser Gln Asn Leu Leu
145                 150                 155                 160
His Ser Asp Gly Lys Thr Tyr Leu Tyr Trp Tyr Leu Gln Lys Pro Gly
                165                 170                 175
Gln Pro Pro Gln Leu Leu Ile Tyr Glu Val Ser Asn Arg Phe Ser Gly
            180                 185                 190
Val Pro Asp Arg Phe Ser Gly Ser Gly Ser Gly Thr Asp Phe Thr Leu
        195                 200                 205
Lys Ile Ser Arg Val Glu Ala Glu Asp Val Gly Val Tyr Tyr Cys Met
    210                 215                 220
Gln Ser Ile Gln Leu His Ser Phe Gly Gln Gly Thr Lys Val Glu Ile
225                 230                 235                 240
Lys Arg

(2) INFORMATION FOR SEQ ID NO:15:

(i) SEQUENCE CHARACTERISTICS:
(A) LENGTH: 726 base pairs
(B) TYPE: nucleic acid
(C) STRANDEDNESS: double

(D) TOPOLOGY: linear

(xi) SEQUENCE DESCRIPTION: SEQ ID NO:15:

ACGTTTGATT TCCACCTTGG TCCCTTGGCC GAACGAGTGA AGCTGTATAC TTTGCATGCA 60
GTAATAAACC CCAACATCCT CAGCCTCCAC CCGGCTGATT TTCAGTGTGA AATCTGTCCC 120
TGACCCGCTG CCACTGAACC TATCTGGCAC TCCAGAGAAC CGGTTGGAAA CTTCATAGAT 180
CAGGAGCTGT GGAGGCTGGC CTGGCTTCTG CAGGTACCAA TACAAATAGG TCTTTCCATC 240
ACTATGCAGG AGGTTCTGAC TASACTTGCA GGAGATGGAG GCCGGCTGTC CAGGGGTGAC 300
GGACAGAGAG AGTGGAGTCT GGGTCATCAC AATATCAAGT GCACTACCGC CAGAGCCACC 360
TCCGCCTGAA CCGCCTCCAC CACTNGAGAC GGTGACCAGG GTACCTTGGC CCCAAAACCG 420
ASTCCCTCTT GCACAGTAAT ACACGGCCGT GTCTTCAGAT CTCAGGCTGC TCAGCTCCAT 480
GTAGGCTGTG CTCGCGGATG TGTCCCTGGT AATGGTGACT CTGCCCTGGA ACTTCTGTGA 540
ATATTTTGTG TTACCATTGT AAGCGCTGAT CCATCCCATC CACTCAAGCC CTTGTCCAGG 600
GGCCTGTCGC ACCCAGCTGA TACCATAGCT GGTAAAGGTG TAACCAGAAG CCTTGCAGGA 660
GACCTTCACT GAGGCCCCAG GCTTCTTCAC CTCAGCCCCA GACTGCACCA GCTGCACCTG 720
GGCCAT 726

(2) INFORMATION FOR SEQ ID NO:16:

(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:16:

GAGTCATTCT CGACTTGCGG CCGCACGTTT GATTTCCASC TTGGTCCC 48

(2) INFORMATION FOR SEQ ID NO:17:

(i) SEQUENCE CHARACTERISTICS:
(A) LENGTH: 48 base pairs
(B) TYPE: nucleic acid
(C) STRANDEDNESS: single
(D) TOPOLOGY: linear

(xi) SEQUENCE DESCRIPTION: SEQ ID NO:17:

GAGTCATTCT CGACTTGCGG CCGCACCTAG GACGGTCAGC TTGGTCCC 48

(2) INFORMATION FOR SEQ ID NO:18:

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:18:

GCGATGGTTG TTGTCATTGT CGGC 24

(2) INFORMATION FOR SEQ ID NO:19:

(i) SEQUENCE CHARACTERISTICS:
(A) LENGTH: 17 base pairs
(B) TYPE: nucleic acid
(C) STRANDEDNESS: single
(D) TOPOLOGY: linear

(xi) SEQUENCE DESCRIPTION: SEQ ID NO:19:

CAGGAAACAG CTATGAC 17

(2) INFORMATION FOR SEQ ID NO:20:

(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:20:

GGTGCTCTTG GAGGAGGGTG C 21

(2) INFORMATION FOR SEQ ID NO:21:

(i) SEQUENCE CHARACTERISTICS:
(A) LENGTH: 369 base pairs
(B) TYPE: nucleic acid
(C) STRANDEDNESS: double
(D) TOPOLOGY: linear

(ix) FEATURE:

(A) NAME/KEY: Coding Sequence
(B) LOCATION: 325...369
(D) OTHER INFORMATION:

(A) NAME/KEY: None
(B) LOCATION: 244...321
(D) OTHER INFORMATION:

(xi) SEQUENCE DESCRIPTION: SEQ ID NO:21:

TTCACACAGG AAACAGCTAT GACCATGATT ACGCCAAGCT TGCATGCAAA TTCTATTTCA 60
AGGAGACAGT CATAATGAAA TACCTATTGC CTACGGCAGC CGCTGGATTG TTATTACTCG 120
CGGCCCAGCC GGCCATGGCC CAGGTGCAGC TGCAGGTCGA CCTCGAGTGG TGGAGGCGGT 180
TCAGGCGGAG GTGGCTCTGG CGGTAGTGCA CAGGTCCAAC TGCAGGAGCT CGATATCAAA 240

CGGGCGGCCG CACATCATCA TCACCATCAC GGGGCCGCAG AACAAAAACT CATCTCAGAA 300
GAGGATCTGA ATGGGGCCGC ATAG ACT GTT GAA AGT TGT TTA GCA AAA CCT 351
Thr Val Glu Ser Cys Leu Ala Lys Pro
1               5

CAT ACA GAA AAT TCA TTT 369
His Thr Glu Asn Ser Phe
10                  15

(2) INFORMATION FOR SEQ ID NO:22:

(i) SEQUENCE CHARACTERISTICS:
(A) LENGTH: 22 amino acids
(B) TYPE: amino acid
(C) STRANDEDNESS: single
(D) TOPOLOGY: linear

(xi) SEQUENCE DESCRIPTION: SEQ ID NO:22:

Met Lys Tyr Leu Leu Pro Thr Ala Ala Ala Gly Leu Leu Leu Leu Ala
1               5                   10                  15
Ala Gln Pro Ala Met Ala
            20

(2) INFORMATION FOR SEQ ID NO:23:

(i) SEQUENCE CHARACTERISTICS:
(A) LENGTH: 16 amino acids
(B) TYPE: amino acid
(C) STRANDEDNESS: single
(D) TOPOLOGY: linear

(xi) SEQUENCE DESCRIPTION: SEQ ID NO:23:

Ser Ser Gly Gly Gly Gly Ser Gly Gly Gly Gly Ser Gly Gly Ser Ala
1               5                   10                  15

(2) INFORMATION FOR SEQ ID NO:24:

(i) SEQUENCE CHARACTERISTICS:
(A) LENGTH: 26 amino acids
(B) TYPE: amino acid
(C) STRANDEDNESS: single
(D) TOPOLOGY: linear

(xi) SEQUENCE DESCRIPTION: SEQ ID NO:24:

Ala Ala Ala His His His His His His Gly Ala Ala Glu Gln Lys Leu
1               5                   10                  15
Ile Ser Glu Glu Asp Leu Asn Gly Ala Ala
            20                  25

(2) INFORMATION FOR SEQ ID NO:25:

(i) SEQUENCE CHARACTERISTICS:
(A) LENGTH: 16 amino acids
(B) TYPE: amino acid
(C) STRANDEDNESS: single
(D) TOPOLOGY: linear

(xi) SEQUENCE DESCRIPTION: SEQ ID NO:25:

Glu Thr Val Glu Ser Cys Leu Ala Lys Pro His Thr Glu Asn Ser Phe
1               5                   10                  15

## Description

### TECHNICAL FIELD

The present invention relates to a method of preparing antibodies that are specific to an antigen complex. The invention also relates to a method of preparing antibodies specific to the prostate-specific antigen/anti-chymotrypsin (PSA-ACT) complex and the uses of such complex-specific antibodies.

### BACKGROUND ART

Prostate-specific antigen (PSA) is a kallikrein-like serine protease mainly expressed in the human prostate. It is responsible for the proteolysis of the gel-forming proteins in human semen.

Since prostate cancer is the second most fatal cancer next to lung cancer for men, the proposal for some type of screening for men over a certain age for early detection seems compelling. The power of using PSA as a screening test is based on the observation that in the absence of cancer serum, PSA is elevated about 0.3 ng/ml per gram of BPH tissue (benign hyperplasia of the prostate) whereas prostate cancer elevates serum PSA about 3.5 ng/ml per gram of cancer as measured by the Yang ProsCheck polyclonal assay (Stamey et al. (1994) Cancer 74:1665). Consequently, the development of a sensitive and accurate PSA assay should be beneficial to the public.

Measurements of serum concentrations of PSA are widely used in monitoring patients with prostate cancer although increased serum concentrations of PSA have also been reported in benign prostatic hyperplasia (BPH) and secondary to surgical trauma of the prostate. In the bloodstream, the proteolytic activity of PSA is inhibited by the formation of irreversible complexes with several different extracellular serum protease inhibitors such as alpha-1-antichymotrypsin (ACT), protein-C inhibitor (PCI), alpha-2-macroglobulin (AMG), inter-α-trypsin inhibitor inhibitor, and with pregnancy zone protein (PZP) (Zhou et al. (1993) Clin. Chem. 39/12:2483-2491; Christensson A. et al. (1990) Eur J. Biochem. 194:755-763; Stenman et al. (1991) *Cancer Res.* 51:222-226). The major PSA inhibitors in the blood are the liver-derived ACT and AMG occuring at concentrations in excess of the normal concentration of PSA in the serum.

The predominant immunodetected form of PSA in serum is complexed to ACT (Lilja et al. (1991) Clin. Chem. 37:1618-1625; Stenman UH et al. (1991) *Cancer Res.* 51:222-226) but PSA exists also in a free, non-complexed form despite the large excess of inhibitors. According to Stamey et al. ((1994) Cancer 74:1665), on average, 88-98% of all PSA recognized by four different commercial assays (listed below) in serum of patients with prostate cancer is complexed to ACT whereas in BPH, the 90-95 kilodalton complexed form represents about 73-84% of total PSA.

Anti-PSA antibodies have been described (see e.g., Stenman UH et al. *Cancer Res.* 51:222-226 (1991); Lilja H. et al. *Clin. chem.* 37:1618-25 (1991); Leinonen J. et al. *Clin. Chem.* 39: 2098-2103 (1993); Christensson A. *J. Urol.* 150:100-5 (1993); Pettersson et al.((1995) Clin, Chem. 41/10: 1480-1488). Although PSA is not a large molecule (MW 34,000 daltons), multiple epitopes can be found. Most of the anti-PSA antibodies available commercially were prepared against the free PSA molecule. Because certain sites on the PSA molecule may be blocked when bound to ACT, it is important not to select antibodies reacting against those sites unless the intention is to set up an assay specific for the free PSA. Commercial assays for PSA include Hybritech Tandem-E and Tandem-R PSA assay kits from Hybritech (San Diego, CA), the Yang ProsCheck (Yang Labs, Bellevue, WA), the Abbott IMx (Abbott laboratories, Abbott Park, IL) and the Ciba-Corning Automated Chemiluminescence System PSA (Ciba-Corning, East Walpole, MA). One of the major problems of many currently used PSA kits is related to the anti-PSA antibodies used. The anti-PSA antibodies employed in these kits not only differ from each other in affinity for both the PSA-ACT complex and free PSA, but their antibodies also may react with these two forms of PSA at different affinities. These different commercial assays have been reported to show discrepancies in PSA measurements (Zhou et al. (1993); Stamey et al., (1994) Cancer 74:1665). For example, according to Stamey et al., (1994) Cancer 74:1662-1666), the Ciba-Corning ACS assay reads 4 times as much free PSA as does the Hybritech assay. These discrepancies in PSA measurement between commercial test systems have been attributed to the differences in recognition of the multiple forms of PSA by reagent antibodies (Zhou et al., 1993).

The existing commercial assays generally involve the capture of free PSA using an antibody directed to free PSA antibody, followed by the addition of an ACT specific antibody to attempt to assess how much of the captured PSA is in the form complexed to PSA. This assay format does not allow wash off of uncomplexed PSA. The current assays require an intermediate wash step prior to the addition of the anti-ACT antibody. This adds to the time required to complete an assay and reduces instrument through-put. Some instruments do not have the capacity to perform the intermediate wash and sequential sandwich ELISA cannot be readily performed.

Most of the previously described antibodies recognize and bind to the PSA portion and do not distinguish between free and complexed PSA. For example, Wu et al. ((1995) Journal of Clinical Laboratory Analysis 9:252-260), tested various commercial monoclonal anti-PSA antibodies and found that they bound both free PSA and PSA-ACT complex. These antibodies may be useful in an assay for total PSA; however, they would not be appropriate for determining the amount of PSA-ACT complex alone. Lilja et al. in U.S. Patent No. 5,501,983 describes a non-competitive immunoassay to measure free PSA and PSA complex. The anti-PSA monoclonal antibodies were produced by the conventional method of immunizing Balb/c mice with PSA and preparing hybridomas. Of the 3 monoclonal antibodies that were characterized for epitope specificity, only one recognized both free PSA and PSA-ACT. The previously described anti-PSA complex antibodies recognize ACT in a complex but are not specific for the PSA/ACT complex in particular. ACT also forms complexes with cathepsin G (Heidtmann and Havemann (1993) Clin. Chem. 39:869-874), chymotrypsin and DNA. Therefore, in PSA assays using such non PSA-ACT complex specific antibodies, the increased absorbance readings may not be exclusively attributed to PSA-ACT since other types of ACT complexes may be present in plasma. Pettersson et al. reported that major difficulties were observed with the five PSA-ACT assays, which all severely overestimated the concentration of PSA-ACT in serum, and proportionately more so at the lower concentrations of PSA, because of non-specific absorbance of ACT or cathepsin G-complexed ACT to the solid phase (Pettersson et al. (1995) Clin, Chem. 41/10: 1480-1488). Pettersson acknowledged that nonspecific and variable interference in the measurements of PSA-ACT will compromise the potential to discriminate between BPH and prostate cancer especially in the clinically important PSA concentrations < 10µg/ml. Yet other PSA antibodies which react with both free and complexed forms do not show equimolar binding to both forms, i.e., the antibody has a different affinity for the different forms of PSA (as exemplified by antibody 2E9 in Pettersson et al. (1995) Clin, Chem. 41/10:1486) and therefore may not provide an accurate measurement of the different PSA forms. Assays using polyclonal antibodies have been found to lose signal when PSA is measured in serum where most of the PSA is complexed to ACT (see Stamey, T. et al. (1994) Cancer 74:1662-1666).

Another factor that may have hindered previous efforts to generate antibodies directed specifically to the PSA-ACT complex is the assay conditions under which PSA/ACT is employed as antigen. Heretofore, efforts to generate antibodies to PSA/ACT complex have tended to rely on the use of the PSA/ACT complex in the various steps of the antibody screening assays, at room temperature in PBS buffer at pH 7.4. These conditions do not favor the stability of the PSA-ACT complex in vitro (Pettersson et al., (1995) Clin. Chem 41/10: 1480-1488).

The present invention overcomes the limitations of the prior art and provides the tools for isolating difficult to obtain complex-specific antibodies.

### SUMMARY OF THE INVENTION

The invention provides a method for obtaining an antibody specific for an antigen complex comprising two or more subunits. In one embodiment where the antigen complex comprises a first subunit and a second subunit, the method comprises the following steps of:
a) providing a library of antibodies displayed by a recombinant replicative vector particle;
b) selecting vector particles from the library that display antibody specific for the antigen complex alone, by contacting the library with the complex and recovering the vector particles that bind the complex;
c) removing vector particles from the library that display antibody specific for the first subunit alone, by contacting the library with the first subunit and recovering the unbound particles;
d) removing vector particles from the library that display antibody specific for the second subunit alone, by contacting the library with the second subunit and recovering the unbound particles;
e) clonally replicating particles that are present in the library after processing according to steps b), c), and d); and
f) selecting clones of replicating particles obtained following step e) that express antibody that binds the complex but not the first or second subunit alone.

The library that remains after step d), can be enriched for vector particles that display antibody specific for the complex alone by further contacting the vector particles with the antigen complex and recovering the vector particles that bind the complex.

The recombinant replicative vector particle can be ay vector particle/organism suitable for library expression and polypeptide display. In one embodiment, the vector particle is a filamentous phage.

It is preferred that the antibody library used in the above method be a naïve antibody library having a large V region repertoire and represented by at least 10⁸ members.

The antibodies can be displayed by the library as an intact antibody or antibody fragment of any form. In one embodiment, the antibodies displayed by the library are single chain variable regions (scFv). Following the selection and isolation of clones of replicating particles that express complex binding antibody in step f), the heavy and light chain variable regions displayed by the selected particle can be further expressed on separate polypeptide chains that reassemble to form a variable region that binds the complex.

It is preferred that the antigen complex be prepared and contacted with the library under conditions that favor the stability of the complex in vitro.

One specific aspect of the invention is a method of obtaining an antibody to a complex according to the preceding embodiments, wherein the first subunit is prostate specific antigen and the second subunit is anti-chymotrypsin, i.e., the complex is PSA-ACT. In this particular method, the antibody library is preferably a naïve human antibody library. For isolating antibodies to the PSA-ACT antigen complex, the complex will preferably be prepared and contacted with the library at 4°C and pH 6.0.

Antibodies prepared by the preceding methods are also provided by the invention. In one embodiment, the heavy and light chain variable region genes are isolated from the vector particle selected for expressing and displaying antibody specific to the antigen complex, and used to recombinantly produce a derivative antibody. The derivative antibody expressing the VH and VL genes isolated from the vector particle can be an antibody in the form selected from the group consisting of Fab fragment, intact antibody, fusion antibody, and chimeric antibody.

One particular complex-specific antibody produced by the preceding methods is an antibody that binds a complex formed between prostate specific antigen (PSA) and anti-chymotrypsin (ACT) with an affinity at least 10 fold higher thin the affinity for either PSA or ACT alone. Preferably, the antibody binds the PSA-ACT complex with an affinity at least 10³ fold higher than the affinity for either PSA or ACT alone. In other preferred embodiments, the antibody specific to PSA-ACT binds the complex with an affinity at least 10 fold higher than the affinity for a complex between other serine proteases and ACT, in particular, between chymotrypsin and ACT.

The invention provides a human scFv antibody directed to PSA-ACT. In another embodiment, the heavy and light chain variable regions of the PSA-ACT antibody are present on separate polypeptide chains. The invention specifically provides the following PSA-ACT antibodies: ITA2 (identified by the amino acid sequence shown in SEQ ID NO. 2); ITA3 (identified by SEQ ID NO. 5); ITA7 (identified by SEQ ID NO. 8); BIOA8 (identified by SEQ ID NO. 11); and BIOC7 (identified by SEQ ID NO. 14). Also provided by the invention are PSA-ACT antibodies wherein the heavy and light chain variable regions or the complementarity determining regions (CDRs) are derived from one of the following antibodies: ITA2, ITA3, ITA7, BIOA8, or BIOC7.

Yet another aspect of the invention is a method of using the aforementioned antibody that binds a PSA-ACT complex with an affinity at least 10 fold higher than the affinity for either PSA or ACT alone, for determining the presence or amount of complex between PSA and ACT in a sample. The method comprises preparing a reaction mixture comprising the antibody and the sample under conditions that permit a PSA-ACT complex to bind the antibody, and determining the presence or amount of any PSA or ACT bound to the antibody in the reaction mixture. This method of measuring the amount of complex between PSA and ACT in a sample obtained from the patient, such as a serum sample, can be applied to a method for distinguishing between a benign and malignant prostate condition in a patient, wherein the amount of the complex is correlated with non-malignancy of the condition. In a separate embodiment, a method is provided for distinguishing between a benign and malignant prostate condition in a patient which involves determining the amount of total PSA in a sample obtained from the patient, measuring the amount of complex between PSA and ACT in that sample according to the preceding embodiments, and correlating the ratio of the complex to the total PSA with non-malignancy of the condition.

### BRIEF DESCRIPTION OF THE DRAWINGS

**Figure 1** is a bar graph depicting phage binding to bio-PSA/ACT versus bio-ACT presented on streptavidin coated plate, as described in Example 1.
**Figure 2** is a bar graph depicting phage binding to PSA/ACT versus PSA as described in Example 1.
**Figure 3** is a bar graph depicting sFv binding to PSA/ACT versus ACT, as described in Example 1.
**Figure 4** is a bar graph depicting sFv binding to PSA/ACT versus CT/ACT, as described in Example 1.
**Figure 5** shows the structure of the phagemid vector pHEN2 used to construct the scFv fragments of the Griffin.1 library as described in Example 1.

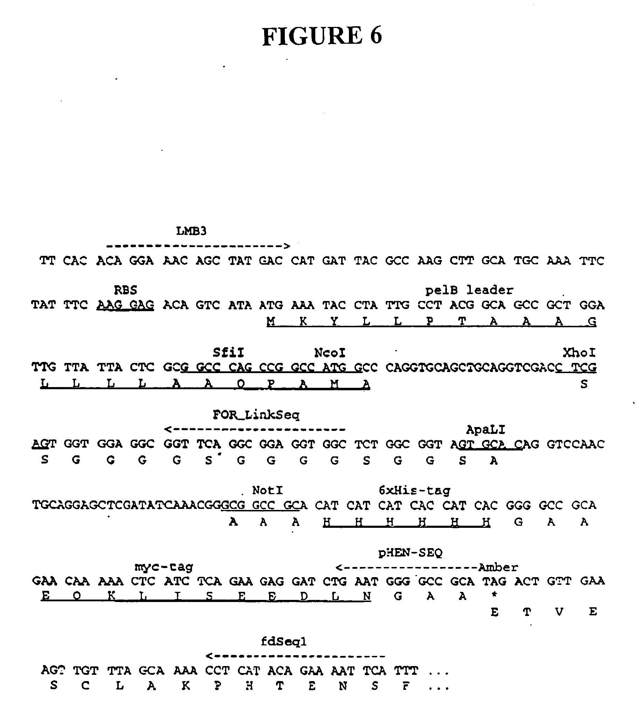

**Figure 6** shows the relevant sequences within the pHEN2 phagemid vector from the LMB3 to the fdSeq 1 site (DNA: SEQ ID NO:21; amino acid sequences: SEQ ID NOS:22-25).

### DETAILED DESCRIPTION FOR CARRYING OUT THE INVENTION

### References

The practice of the present invention will employ, unless otherwise indicated, conventional techniques of molecular biology and the like, which are within the skill of the art. Such techniques are explained fully in the literature. See e.g., Molecular Cloning: A Laboratory Manual, (J. Sambrook et al., Cold Spring Harbor Laboratory, Cold Spring Harbor, N.Y., 1989); Current Protocols in Molecular Biology (F. Ausubel et al. eds., 1987 and updated); Essential Molecular Biology (T. Brown ed., IRL Press 1991); Gene Expression Technology (Goeddel ed., Academic Press 1991); Methods for Cloning and Analysis of Eukaryotic Genes (A. Bothwell et al. eds., Bartlett Publ. 1990); Gene Transfer and Expression (M. Kriegler, Stockton Press 1990); Recombinant DNA Methodology II (R. Wu ed., Academic Press 1995); PCR: A Practical Approach (M. McPherson et al., IRL Press at Oxford University Press 1991); Cell Culture for Biochemists (R. Adams ed., Elsevier Science Publishers 1990); Gene Transfer Vectors for Mammalian Cells (J. Miller & M. Calos eds., 1987); Mammalian Cell Biotechnology (M. Butler ed., 1991); Animal Cell Culture (J. Pollard et al. eds., Humana Press 1990); Animal Cell Culture (R. Freshney ed., IRL Press 1987); Flow Cytometry and Sorting (M. Melamed et al. eds., Wiley-Liss 1990); the series Methods in Enzymology (Academic Press, Inc.); Techniques in Immunocytochemistry, (G. Bullock & P. Petrusz eds., Academic Press 1982, 1983, 1985, 1989); Handbook of Experimental Immunology, (D. Weir & C. Blackwell, eds.); Cellular and Molecular Immunology (A. Abbas et al., W.B. Saunders Co. 1991, 1994); Current. Protocols in Immunology (J. Coligan et al. eds. 1991); and the series Annual Review of Immunology; the series Advances in Immunology; Oligonucleotide Synthesis (M. Gait ed., 1984).

Additional references describing the preparation of phage and phagemid display libraries for human antibodies, synthetic antibodies, codon mutagenesis, single-chain Fv design and production, etc., which may be used in the methods of the present invention include the following: Antibody Engineering, 2^{nd} edition (C. Borrebaeck, ed., Oxford University Press, 1995); Marks, J.D. et al. (1991) J. Mol. Biol. 222:581-597; Griffiths, AD et al. (1993) EMBO J. 12:725; Griffiths, A.D. et al. (1994) EMBO J. 13:3245-3460. Additional references providing protocols for the various immunoassay formats include Immunoassay (E. P. Diamandis & T.K. Christopoulos, eds., Academic Press, Inc., 1996).

The references cited in the above section are hereby incorporated by reference herein to the extent that these references teach techniques that are employed in the practice of the present invention. In addition, other references cited within this application, including patents, published applications and other publications, are hereby incorporated by reference.

### Definitions

A "complex" or "antigen complex" of the present invention consists of at least 2 subunits in association either covalently or non-covalently. PSA/ACT is described herein as an exemplary complex which consists of two subunits, PSA and ACT, but complexes involving 3 or more subunits are encompassed by the methods of the invention. The complex as well as the subunits that make up the complex are not limited to any molecular size, structural or compositional feature.

A "subunit" of an antigen complex can be a protein, peptide, lipid, sugar, cofactor, hormone, toxin, drug, or any compound from plant or animal source. The subunits can interact or associate in any manner to form the complex.

Conditions that affect the stability of the purified antigen complex in vitro include temperature, pH, type of buffer, salt conditions, the concentration of each subunit, the presence of cofactors and protease inhibitors. In the present method of isolating complex-specific antibodies, the antigen complex when used in vitro (such as in the antibody library selection and screening assays) will preferably be under conditions that favor or maximize the stability of the complex without adversely affecting the stability of the other components of the assay. For example, in the particular example of preparing antibodies to the PSA-ACT complex, the antigen complex is maintained under pH and temperature conditions that favor its in vitro stability but are also compatible with the stability and function of the phage particles in the assays. Preferably, the antigen complex is stable in vitro for at least the duration of each of the selection and screening assays. The conditions that favor the stability of the complex will vary with the antigen but can be readily determined by one of skill in the art such as by systematically varying the different parameters, one at a time and then in combination and measuring the amount of complex with time.

The term "antibody" as used throughout this application is defined to include not only intact antibody molecules, but any molecule comprising at least one variable region or portion with the desired binding specificity. The variable region will typically comprise a V_{H}-V_{L} pair, but may alternatively be made up of other combinations of variable chains from antibodies or T cell receptors. Variable region fragments, naturally occurring or synthetic variants, fusion molecules, chimeras, mosaics, and humanized variants are included, so long as the binding specificity is retained. The variable region will be presented in any suitable form, including but not limited to intact antibody molecules and antibody fragments (such as Fab, F(ab')₂, Fd and Fv and other antigen-binding fragment). Examples of constructs of particular interest include single chain variable region polypeptides (scFv), in which a single V_{H}-V_{L} pair are linked through a flexible peptide linker sequence to form a single polypeptide chain in a manner that permits the polypeptide to fold into the three-dimensional conformation of a single variable region, and diabodies, which is a polypeptide dimer with two V_{H}-V_{L} sites. "scFv" and "sFv" both refer to single chain variable region polypeptides and are used interchangeably herein. Chimeric antibodies consist of immunoglobulin V regions from a different species (most often rodent) linked to human C regions. Mosaic antibodies are composed of humanized V regions (usually rodent CDRs grafted onto human frameworks) and human C regions. Antibody fusion molecules will comprise any of the aforementioned forms of antibody fused to a non-antibody entity typically at a site that does not interfere with the binding of the antibody to the target antigen complex. The non-antibody entity can be a tag such as an epitope tag (e.g., c-myc peptide, influenza virus hemagglutinin (HA)), a member of a binding pair (e.g., avidin), an enzyme (e.g. horse radish peroxidase, alkaline phospahatase, β-galactosidase), a cleavable sequence (e.g., phosphatidylinositol-glycan or PIG sequence) or any suitable entity. For the construction of these various forms of antibodies, refer to, e.g., Antibody Engineering, 2^{nd} edition (C. Borrebaeck, ed., Oxford University Press, 1995) and Immunoassay (E. P. Diamandis & T.K. Christopoulos, eds., Academic Press, Inc., 1996).

A "naive" antibody library is a library created from an mammal which does not have a preponderance of antibodies to a single immunogen. A naïve antibody library is prepared in a manner so as to eliminate or minimize selection pressure and somatic mutation on the immunoglobulin repertoire that would result in deletion of self-reactive B-cell clones. An antibody library is considered a "naïve" library when prepared from a mammal that has not been specifically immunized or challenged with antigen.

The antibody library will preferably contain a sufficiently large and diverse V region repertoire. The V region repertoire will comprise at least 10⁷ different members, preferably at least 10⁸, even more preferably at least 10¹⁰ members.

A "recombinant replicative particle" as used herein can be a phage, bacteria, yeast, insect cell or mammalian cell. Preferably the recombinant replicative particle is a filamentous phage such as M13 or fd phage. If the vector is filamentous phage, the antibody fragments can be fused to the phage minor coat protein (gene III protein) or the major coat protein.

A "phagemid" is a plasmid that includes a phage origin of replication.

### Detailed description of preferred embodiments

The invention provides a method for obtaining an antibody specific for an antigen complex composed of two or more subunits. The method relies on the screening of a large naïve antibody library and also on the use of the antigen complex in the various screening steps under conditions that favor the stability of the complex. By starting with a large and naïve antibody repertoire, this method increases the probability of isolating antibodies that are difficult to obtain using conventional procedures involving animal immunization or antibody libraries derived from immunized animals. In particular, this method circumvents the problems associated with obtaining antibodies to "self antigens" and to antigens of the lumen of the endoplasmic reticulum. Animals do not normally produce antibodies to self antigens and vaccination of an animal with its self antigen does not normally raise antibodies specific to the antigen due to clonal deletion and anergy leading to self tolerance.

The method of the invention can be used to generate a antibody specific to any antigen complex including enzyme/enzyme inhibitor complexes such as PSA/ACT or thrombin/anti-thrombin. In one embodiment, the method of the invention is used to generate an antibody specific to a complex composed of the subunits, prostate-specific antigen (PSA) and anti-chymotrypsin (ACT). The complex is abbreviated herein as PSA-ACT or PSA/ACT.

Antibodies to antigen complexes prepared by the presently disclosed methods are also provided. Complex specific antibodies have may uses, for example, in immunoassays, diagnosis, immunotherapy, affinity chromatography, antigen complex screening or detection, isolation and purification.

In an exemplary antigen complex that comprises two subunits, the method comprises the following steps:
a) providing a library of antibodies displayed by a recombinant replicative vector particle;
b) selecting vector particles from the library that display antibody specific for the antigen complex alone, by contacting the library with the complex and recovering the vector particles that bind the complex;
c) removing vector particles from the library that display antibody specific for the first subunit alone, by contacting the library with the first subunit and recovering the unbound particles;
d) removing vector particles from the library that display antibody specific for the second subunit alone, by contacting the library with the second subunit and recovering the unbound particles;
e) clonally replicating particles that are present in the library after processing according to steps b), c), and d); and
f) selecting clones of replicating particles obtained following step e) that express antibody that binds the complex but not the first or second subunit alone.

For the construction of large human antibody library from unimmunized donors (naïve library), see Example 1 herein as well as Griffiths, A.D. et al., (1994) EMBO J. **13**: 3245-3260, Vaughan, T.J. et al. (1996) Nature Biotechnology 14(3): 309-314, and in WO 93/11236 (Griffiths et al), all incorporated herein by reference. The naïve library is prepared from unimmunized mammals. Diverse libraries of human immunoglobulin VH and VL genes are most conveniently prepared from the peripheral blood lymphocytes (PBLs) of nonimmunized donors, using polymerase chain reaction (PCR) amplification of the immunoglobulin genes. Preferably, the library is prepared from a immunoglobulin sequence pool from at least 2 unimmunized individuals, more preferably from at least 20, donors, even more prefereably from 50 or more donors. The greater the number of donors contributing to the pool, the greater the representation of V region genes and antigen specificity.

Highly diverse synthetic repertoires of H and L chains or antibody fragments such as Fab or scFv can be created entirely in vitro from a bank of V gene segments by recombination of the repertoires in, e.g., bacteria. In one embodiment, the antibodies displayed by the library are single chain variable region fragments or scFv. To generate the genes encoding scFv fragments, the VH and VL genes can be randomly combined using PCR and the combinatorial library cloned for display on the surface of a replicative vector particle such as a filamentous phage. Synthetic libraries created, for example, by in vitro rearrangement of germ line V regions and random combinations of VH and VL further increase the diversity of the antigen binding repertoire. Random sequences of CDR loops can also be created. Methods for the preparation of synthetic antibodies, scFv, humanized antibodies, and the engineering of antibody combining site by codon-based mutagenesis are described in the art, see e.g., Antibody Engineering, Borrebaeck, C. (ed). 1995, Griffiths et al. 1994, ibid., Marks et al. 1991, ibid.

It is desirable to employ a "large" antibody library having V region repertoires that are extremely diverse in sequence. The library preferably represents at least 10⁷ different members, more preferably at least 10⁸, or even more preferably 10¹⁰ or greater members.

The antibody library can be constructed using immunoglobulin genes from any species but preferably from a mammal such as a rodent, rabbit, goat, sheep, horse, pig, cow, human, etc. Where the complex-specific antibody is intended for diagnostic use involving human samples such as serum or other human biological fluids, it is preferable that the antibody be a human or humanized antibody so as to minimize any undesirable cross-species reactivity with other components of the human sample. In that case, it is desirable that a human or humanized antibody library be screened. Likewise, it is preferable to use a human, humanized or chimeric human antibody library to isolate complex antibodies intended for therapeutic applications in humans. Alternatively, the immunoglobulin genes from a non-human antibody library found to encode complex-specific antibodies upon screening, can be isolated from the vector particle and used to prepare second generation derivatives which are humanized, chimeric or mosaic.

The Griffin.1 scFv phagemid library used herein to isolate antibodies directed to PSA-ACT is a library made up of germ-line heavy and light chain variable region genes in random VH-VL dimers, yielding a repertory of about 10¹³ combinations. The V regions from the library described in Griffiths, A.D. et al., (1994) EMBO J. **13**: 3245-3260 were subcloned and assembled into scFv to form the Griffin.1 scFv library.

### Antibody Library Screening and Selection Strategies

Described below is the general scheme for the screening and isolation of complex specific antibodies. The experimental examples describe the methods in further detail. For convenience, the antibody library will be discussed herein using phage display library as an example. However, it should be understood that the antibody library need not be presented on phage but can be presented using other suitable polypeptide display systems.

A naïve antibody library is screened for antibodies that bind the antigen complex. The screening strategy involves a combination of both a) positive selection to select for the complex-specific antibodies and; b) negative selection or "subtraction" to remove antibodies to the free subunits that mike up the complex. It is preferable that the selection be performed in the order of positive selection first followed by negative selection. Following the subtraction rounds, another positive selection is preferable but optional. Several rounds of each type of selection are recommended, preferably at least 2-3 rounds. Each round of selection involves panning and amplification (growth). In the case of phage libraries, the positively selected phage that are bound to the antigen complex are eluted ad the phage amplified clonally in a bacterial host. By rounds of selection and growth, rare antigen-binders are selected.

The panning and amplification steps are known in the art and are exemplified below in the experimental Examples. Selection can be performed in solid phase or soluble phase or a combination of both. Suitable solid phase matrices for carrying out the selection procedure include test tubes (such as immunotubes) and plastic plates.

The preparation of the antigen complex will vary with the chemical, physical and biological properties of each antigen. The complex will generally be prepared in a solution that is compatible with the conditions for the screening and imunoassays and under conditions that favor the stability of the complex in vitro.

### Isolation of positively selected vector particles and antibody sequences

At the end of the selection process, the vector particles that have been selected for expression of antibody that bind the antigen complex are replicated by growing the particle. The sequences encoding antibody having the desired specificity can then be isolated from the vector particle and subcloned for expression as soluble antibodies in bacteria, eukaryotic cells or other suitable expression systems that will retain the antigen binding ability of the antibody or antibody fragment. For example, the antibody sequences isolated from a phagemid clone can be used to transform bacteria to produce soluble antibody in the bacterial periplasm. The soluble antibody is then tested for specific binding to the antigen complex.

Sequencing can be performed to analyze the sequences encoding the complex-specific antibody, specifically the V regions. The VH and VL displayed by the selected vector particle can be expressed on separate polypeptide chains that reassemble to form a V region that binds the complex. The isolated sequences can be further subjected to site directed mutagenesis and/or other recombinant techniques to modify the sequences to improve the binding affinity of the antibody, to correct mutations (e.g. amber mutations) and to generate derivatives of the antibody to specifically tailor the antibody for particular uses. For example, Fab, Fd, scFv antibody fragments, intact antibody, fusion, chimeric, mosaic or humanized antibodies can be further generated from the antibody sequences isolated from the selected vector particle.

### Preparation of PSA-ACT complex specific antibodies

The use of vector display technology in the present method of preparing an antibody to the PSA-ACT complex bypasses many of the problems of animal immunization. A library of synthetic human single chain Fv fragments (Griffin.1 scFv phagemid library) was selected using solid phase PSA/ACT complex coated on immunotubes, and also biotinylated PSA/ACT complex (biotinylated via lysine residues or carbohydrate) and streptavidin-coated magnetic beads. Each selection strategy involved depleting the selected library of phages binding only to PSA or to ACT alone. In addition, the selections were specifically performed at 4°C and pH 6 to favor the stability of PSA/ACT complex in vitro. These use of a large naïve antibody library as well as the use of the PSA-ACT complex in vitro at 4°C and pH 6 in the screening likely played a major role in enabling the present isolation of PSA/ACT antibodies which do not cross react with ACT complexed to other entities.

The PSA-ACT complex is preferably prepared, stored and used at a temperature of less than 35°⁰C, preferably at 4°C, and at a pH of between 6.0 to 7.4, preferably at pH 6.0. The addition of 100-1000 fold molar excess of native ACT may also add to the stability of the complex although it was not necessary in the present methods. In the methods of isolating PSA-ACT complex-specific antibodies of the present invention, all the screening steps including the coating of microtiter plates and tubes, incubations, and wash steps were performed at 4°C and pH 6. PSA and ACT used in the methods of the present invention can be prepared, for example, as described in Christensson A. et al. (1990) Eur. J. Biochem. 194:755-763.

The invention provides an antibody that binds a complex formed between prostate specific antigen (PSA) and anti-chymotrypsin (ACT) with no or insignificant binding to PSA and ACT alone. In a preferred embodiment, the antibody is human antibody or one comprising human V region sequences.

Preferably, the antibody binds the complex with an affinity of at least 5 fold, preferably 10-10², even more preferably, at least 10³ fold higher than the affinity for either PSA or ACT alone. In one embodiment, the antibody binds PSA-ACT with an affinity at least 10 fold higher than the affinity for either PSA or ACT alone. In another embodiment, the antibody binds the complex with an affinity at least 10 fold higher than the affinity for a complex between other serine proteases and ACT. In one particular embodiment, the antibody binds PSA-ACT with an affinity at least 10 fold higher than the affinity for the complex between chymotrypsin and ACT (CT-ACT). High affinity, preferably less than 10 nM, complex antibodies are desirable.

The following antibodies to PSA-ACT were obtained from selected phage particles using the preceding method: ITA2, ITA3, ITA7, BIOA8, and BIOC7. The nucleotide and amino acid sequences (VH-linker-VL) of each of these scFv antibodies are shown in the sequence listing below: ITA2 (SEQ ID NO. 1-3); ITA3 (SEQ ID NO. 4-6); ITA7 (SEQ ID NO. 7-9); BIOA8 (SEQ ID NO. 10-12); AND BIOC7 (SEQ ID NO. 13-15). The sequences for each antibody are in the order of DNA nucleotide sequence, amino acid sequence and the sequence of the DNA complementary strand.

### Applications of antibodies to PSA-ACT complex

Antibodies to PSA/ACT complexes produced by the methods of the present invention have uses to determine the presence and the amount of PSA/ACT complexes in a sample, in particular, diagnostic uses. The availability of PSA-ACT complex specific antibodies allows the capture of only the antigen of interest and enables the development of more precise, sensitive and faster diagnostic assays for measuring PSA-ACT complexes in patient samples.

The individual undergoing diagnosis can be a patient suspected of suffering from a disease condition in which the levels of PSA/ACT are elevated above that in the normal, benign or non-diseased state. It has been recommended by both the American Cancer Society and the American Urological Association that all men over 50 years of age should receive an annual examination, consisting of a DRE and a serum PSA test, for the detection of early prostate cancer. Measuring the serum concentration of PSA is useful for the early detection (screening) of prostate tumors as well as for the management of patients with prostate cancer, e.g., for monitoring patients during radio- and chemotherapy, to assess the success of a prostatectomy, the disclosure of metastasis, and allows the detection of recurrence of cancer after surgery at much earlier stages. For monitoring recovery from prostate cancer after chemotherapy, it would be helpful to have the serum PSA levels before and at various times during as well as after treatment. For screening male subjects for prostate cancer, it is preferable that yearly determination of PSA levels be combined with digital rectal examination to improve the rate of early detection of prostate cancer.

The PSA diagnostic assay employing the PSA-ACT antibodies of the present invention will generally be sensitive to detect PSA-ACT at low concentration ranges, such as between the levels of 0-20 ng/ml as well as elevated concentrations up to and above 4 µg/ml of PSA.

Thus, the invention provides a method for determining the presence or amount of complex between PSA and ACT in a sample that makes use a PSA-ACT complex specific antibody produced by the methods of the invention. The method involves preparing a reaction mixture comprising the antibody and the sample under conditions that permit a PSA-ACT complex to bind the antibody, and determing the presence or amount of any PSA or ACT bound to the antibody in the reaction mixture.

The sample will typically be but is not limited to, a liquid sample, including biological fluids ad solubilized tissue samples. Exemplary biological fluids include but is not limited to serum, plasma, seminal fluid and semen.

The various configurations of immunoassays (competitive and noncompetitive) suitable for carrying out the measurements of PSA-ACT complexes will be familiar to the skilled artisan and are described, for example, in Diamandis et al. (1996), Immunoassay, ibid. The preferred assay is one that provides a low background, consistent and reproducible results, efficiency and ease of performance. Preferably, the assay is an ELISA assay, in a sandwich format an can be automated or semi-automated. A preferred method of assaying for PSA-ACT complex using the antibodies of the invention is by the use of the automated ELECSYS® immunoassay system available from Boehringer Mannheim Corporation. The Elecsys® immunoassay system is based on electrochemiluminescence (ECL) detection and uses the ruthenium(II)-tris(bipyridyl) complex as the ECL label. Briefly, the system works on the following principle. Paramagnetic microparticles are coated with streptavidin. A biotin-labeled antibody and a ruthenium-labeled antibody (for the PSA assays of the present invention, these labeled antibodies would be anti-PSA antibodies) are incubated with a sample analyte, e.g., PSA-ACT. The immune complex is captured by the streptavidin-coated microparticles which have high biotin binding capacity. Inside the ECL measuring cell, the microparticles with their bound immune complexes are uniformly deposited on an electrode and the unbound components are washed away at the end of the incubation, in a single wash step. The bound sample analyte is quantitated by applying a voltage to the electrode during which the ECL label releases a photon that can be measured as light. Once the ECL reaction is completed, the magnetic microparticles are released from the electrode surface and washed away. The electrode surface is thoroughly cleaned and the cell is ready for another measurement. The immunoassay is automated and computer-based and can be run on an Elecsys 1010 or the greater capacityr 2010 system machine. This system provides prompt results for patient diagnosis as the incubation time for most Elecsys® immunoassays is only 9-18 minutes. The Elecsys® system is currently used clinically to measure serum concentrations of thyroid stimulating hormone (TSH) in screening for hyperthyroidism and hypothyroidism, hCG infertility assays, and troponin T and CK-MB in assessing risk in patients with acute myocardial ischemia (CARDIAC T® assay).

The PSA-ACT diagnostic assay can be performed in the Elecsys® system as a non-competitive sandwich assay as described above with a PSA-ACT antibody, biotin labeled and ruthenium labeled. The amount of ECL light signal measured will correlate directly with the amount of bound patient PSA-ACT. Alternatively, the assay can be in a competitive format with PSA-ACT from the patient sample competing with ECL-labeled PSA-ACT for the antibody binding site on the immobilized anti-complex antibody. In the competitive format, the ECL signal measured is in inverse relationship with the amount of PSA-ACT.

The PSA-ACT antibodies of the present invention are able to bind PSA-ACT at pH 6.0 and at pH 7.4. The complex antibody can be conjugated to various tags and enzyme markers where necessary to facilitate detection and allow quantitation of antigen bound by the antibody. Methods of conjugating antibodies to various tags are known in the art.

The PSA-ACT antibodies of the invention can be used in a method to distinguish between a benign and malignant prostate condition in a patient by measuring the amount of complex between PSA and ACT in a sample obtained from the patient, according to the preceding embodiments, and correlating the amount of the complex with non-malignancy of the condition. Alternatively, the amount of total PSA and the amount of PSA-ACT complex in a patient sample are measured and the ratio of the complex to the total PSA determined. To measure the amount of total PSA, one or more PSA antibodies can be employed in the assay. The sample obtained from the patient will typically be serum. With the availability of the present antibodies specific to PSA-ACT complex and the existing antibodies that detect free PSA only, these values of free, ACT complexed and total PSA can be readily determined. These values can then be compared with a reference range or calibrator to provide a diagnosis of malignancy or non-malignancy and to assess the progress of prostate disease.

PSA increases with age and gland volume in the absence of prostate cancer (Babaian RJ et al. (1992) J. Urol. 147:837-840). Therefore, it is useful to establish a calibrator or PSA-ACT reference range to allow more accurate correlation of serum PSA measurements to disease conditions. Age-specific reference values for PSA-ACT and free would increase the sensitivity and specificity of diagnosis of prostate cancer. These references values will be most useful if they are either continuous or at very close age intervals. Preferably, the age-specific reference range will be established for randomly chosen healthy men in the 40-80 year age group who do not have clinical evidence of prostate cancer. A PSA-ACT reference may also be set up based on increasing volumes of prostate cancer. The "normal" level of PSA is regarded as 4.0 ng/ml although this level does not take into account age differences in serum PSA level. The values for the amount of PSA-ACT or the ratio of complex to total PSA determined by the methods of the present invention will then be compared with the reference values for meaningful interpretation.

Finally, diagnostic assay kits for prostate cancer are provided that comprise the PSA-ACT complex antibodies of the present invention alone or in combination with one or more antibodies to free PSA to quantitate at least PSA-ACT complex in the patient serum sample.

### EXAMPLES

### Example 1

Example 1 describes the screening for and isolation of scFv antibodies specific to the PSA-ACT complex.
**Strategy:** Detection of Prostate Specific Antigen complexed to anti chymotrypsin (PSA-ACT) could provide a sensitive format for prostate cancer diagnosis. Because the complex is temperature and pH sensitive, the use of a phage display library should be more productive than animal immunization in identifying a complex-specific antibody. Phage display technology also allows the subtraction of fragments which bind either subunit from the complex-specific pool. In addition to being specific for complexed PSA these antibodies need to recognize ACT only when complexed to PSA and not to other serine proteases such as chymotrypsin. Two distinct screening strategies were used here to isolate anti-PSA-ACT binding sFvs.
**Materials:** Free PSA and PSA/ACT complex were obtained from Scripps Laboratories (San Diego CA). Free ACT was purchased from Boehringer Ingelheim Bioproducts (Heidleberg, Germany) and Serva (Crescent Chemical Co., (distributor) Hauppauge, NY). Phage amplification and growth was carried out in E. coli supressor strain TG-1 (K12, Δ(*lac-pro*), *sup* E, *thi, hsd* D5/F' *tra* D36, *pro* A⁺B⁺, *lac* I^{q}, *lac* ZDM15) (Gibson, T.J. (1984) Ph.D. Thesis, University of Cambridge, UK). Generation of sFvs was carried out in TG-1 and in non supressor strain HB2151 ( K12. *ara*, Δ(*lac*-*pro*), *thi*/F' *pro* A⁺B⁺, *lac* I^{q}, *lac* ZΔM15) (Carter, P. et al., (1985) Nucleic Acids Research. **13**:4431-4443.). Phage rescues were performed with helper phage M13 KO7 (Pharmacia). All cell growth utilized 2XTY media (16g Tryptone, 10g yeast extract and 5g NaCl /liter). All binding reactions were performed at 4°C in a acetate buffer (10mM NaOAC, 150mM NaCl pH 6.0) herein referred to as AB-6. Incubations for selection were done on a over-under turntable unless otherwise stated.

### 1.1 Construction of the Griffin.1 antibody library

A large naive human phage-display sFv library referred to herein as the Griffin.1 scFv phagemid library, was used to isolate antibody fragments which will specifically bind the PSA-ACT complex. Not wishing to be bound by any theory, model or mechanism, the possible epitope would be a conformational epitope present only for this complex.

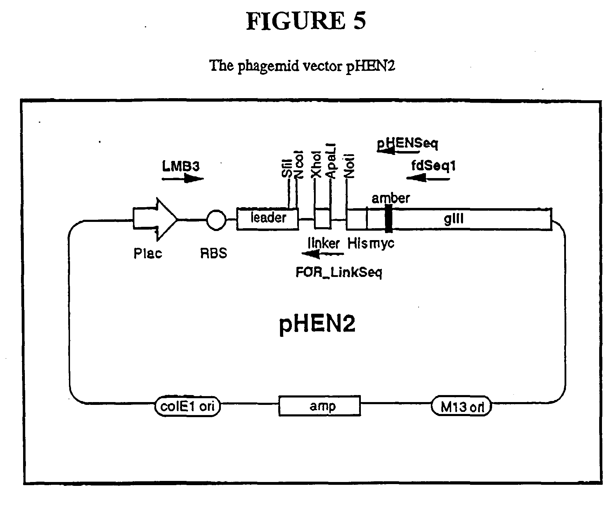

The Griffin.1 library is a scFv phagemid library (Marks JD et al. (1991). By-passing immunization. Human antibodies from V-gene libraries displayed on phage, J Mol Biol, 222: 581-97) which was constructed by recloning the heavy and light chain variable regions from the lox library vectors (Griffiths, AD et al. (1994) Isolation of high affinity human antibodies directly from large synthetic repertoires, EMBO J, 13, 3245-3260) into the phagemid vector pHEN2 (see Figure 5).

The kappa and lambda light chain variable regions were PCR amplified from the fdDOG-2loxVk and VL phage constructs using the primers:-
5'GAGTCATTCTCGACTTGCGGCCGCACGTTTGATTTCCASCTTGGTCCC (SEQ ID NO. 16) or 5'GAGTCATTCTCGACTTGCGGCCGCACCTAGGACGGTCAGCTTGGTCC
C (SEQ ID NO. 17) and
FdPCRback: 5'GCGATGGTTGTTGTCATTGTCGGC (SEQ ID NO. 18).

The PCR fragments were purified and digested with ApaL 1 and Not1. The gel purified fragments were then ligated into the vector pHEN2 in several aliquots. DNA was then purified from the ligation mixtures, resuspended in water, and electroporated into E.coli TG1. Vk-pHEN2 or VL-pHEN2 library pools of 3.5 x 10⁷ and 1.67 x 10⁷ respectivley, were obtained.

Heavy chain variable regions were PCR amplified from the pUC19-2loxVH vector using the primers:-
**LMB3** (5'CAG GAA ACA GCT ATG AC) (SEQ ID NO. 19)and
**CH1.LIBSEQ** (5'GGTGCTCTTGGAGGAGGGTGC) (SEQ ID NO. 20).

The PCR fragments were purified and digested with Sfi 1 or Nco 1 and Xho 1. The gel purified fragments were then ligated into the vectors Vk-pHEN2 or VL-pHEN2. DNA was purified from the ligation mixtures, resuspended in water, and used for several hundred electroporations into E.coli TG1 to obtain a total library size of 2 x 10⁹.

The scFv fragments contain a small c-myc peptide fused at the C-terminus (see Figure 5) as a tag to facilitate detection of the soluble scFv fragment using an anti-c-myc monoclonal antibody conjugated to horse radish peroxidase (HRP).

### 1.2 Selection strategies

### 1.2A Solid phase selection on immunotubes

Immunotube panning, a standard approach to phage display selection was performed as described in Marks et al (1991) except that incubations were carried out at 4°C and instead of PBS we used an acetate buffer-AB-6 (10mM NaOAC, 150mM NaCl pH 6.0) Starting with 1x10¹³ phage from the Griffin.1 library, two rounds of panning were performed on Nunc Maxysorb Immuno test tubes (Fisher) coated overnight with 25µg/ml PSA/ACT in AB-6 and then blocked for at least 1 hr with 2% milk powder/AB-6 (MP/AB-6). Selected phage were amplified in TG-1, rescued with Helper phage M13 KO7 (Pharmacia) and phage particles purified by PEG-precipitation (see phage preparation section-below).

In order to remove phage which bound either PSA or ACT rather than complex-specific epitopes, two further rounds of panning were carried out with the inclusion of "phage-subtraction" prior to selection. Two immunotubes were coated overnight with ACT (free of PSA/ACT) at 50µg/ml, two were coated with free PSA at 25µg/ml and one was coated with PSA/ACT at 25µg/ml. Phage (1x10¹³) from the second round of selection were added to the first ACT coated immunotube and rotated for 30 minutes then transferred to the second ACT-coated tube for an additional 30 minutes on a rotator then allowed to stand for 30 minutes. The phage still in solution were transferred to a PSA-coated tube for 30 minutes with rotation , then a second PSA-coated tube for 60 minutes (30 minutes on a rotator, 30 minutes standing). Finally the phage were added to the PSA/ACT coated tube for a 60 minutes incubation (30 minutes on a rotator, 30 minutes standing). The PSA/ACT tube was then washed as before, the phage eluted with TEA (triethylamine at 100mM), neutralized, and amplified in TG1.

(When it was later found that PSA did not adhere well to plastic, 2 rounds of soluble subtraction using 50nM biotinylated PSA and 500µl of streptavadin beads as described in the next section, were performed on this material , again followed by positive selection on PSA/ACT coated immunotube for 6 total rounds).

### 1.2B. Selection on biotinylated material

The possibility that adherence to plastic might alter PSA/ACT enough to obscure potential complex-specific epitopes led to the inclusion of a soluble phase selection wherein the phage library was incubated with biotinylated-PSA/ACT (Bio-PSA/ACT), and binders collected on magnetic streptavadin-coated particles (Hawkins, R.E., et al., (1992) J. Mol. Biol. 226: 889-896). Two PSA/ACT biotinylations were carried out, one via NH₂₋groups with a challenge ratio of 1:3 (PSA-ACT-BI(XOSu)) the other via carbohydrate groups with a challenge ratio of 1:250 (PSA-ACT(ox)-Bi(HZ)) and they were used as a pool with 50nM of each in the selections. Three rounds of soluble phase selection were performed with the pooled biotinylated material.

In the first round, the library (1x10¹³ phage) was incubated with 100 nM Bio-PSA/ACT in 5 ml of MP/AB-6 for 1 hr at 4°C with rotation. The phage binding to Bio-PSA/ACT were collected for 15 minutes on 1.5 ml of streptavadin magnetic dynabeads M-280 (Dynal, Oslo Norway) previously blocked with 2% MP/AB-6. The beads were washed 15 times with AB-6, every third wash included 2% milk powder. Phage were eluted from the beads with 1 ml 100 mM TEA (10 minutes rotation at room temperature) and neutralized with 0.5 ml Tris-HCl pH 7.4 The beads were also neutralized with 0.5 ml Tris-HCl pH 7.4, and along with 0.75 ml of the eluted phage, used to infect 9 mls TG-1 bacteria. The second selection was collected on 300µl of avidin magnetic beads (CPG, Lincoln Park, N.J.) to avoid carrying streptavadin-binders through our selection. The third round was collected on 300µl of streptavidin magnetic beads.

To allow the subtraction of phage-displayed sFvs which bound either subunit, free PSA and free ACT were each biotinylated, again both at carbohydrate and NH2 groups {(PSA-Bi(XOSu), PSA-Bi(HZ), ACT-BI(XOSu) and ACT-BI(HZ)} and used as a cocktail of 50 nM each. In the subtractive rounds of selection which followed, phage were incubated first with 100nM bio-PSA in 5 mls MP/AB-6 1hr at 4°C and binding phage collected on 2x 0.5 ml streptavidin magnetic beads (15 minutes) and discarded. 100nM bio-ACT was then added to the remaining phage and incubated for 1 hr at 4°C. The binding phage were again removed with 2x0.5 ml streptavidin magnetic beads and discarded. Remaining phage were allowed to bind bio-ACT/PSA ( 100nM 1hr, 4°C), collected on streptavidin beads eluted and amplified. The order of PSA vs ACT was switched in the second round of subtraction.

### 1.3 Screening of selected repertoires.

After each round of selection, phage mixtures from the library were screened by Polyclonal Phage ELISA as described in Griffiths, A.D. et al., (1994) EMBO J. **13**: 3245-3260, except AB-6 buffer was used instead of PBS. Falcon 9312, 96-well Flex micro-assay plates were coated overnight with PSA/ACT, free PSA or free-ACT at 10µg/ml in AB-6. Binding phage were detected with an HRP/Anti-m13 conjugate (from Pharmacia).

The diversity of the phage displayed antibodies after various rounds of selection, was determined by colony PCRs (Gussow, D. & Clackson. (1989) T. Nucleic Acids Research. **17**: 4000) followed by BSTN I digestion and gel analysis.

### 1.4 Screening and sequencing of clones.

Single ampicillin (amp) resistant colonies from selected repertoires were screened for the production of PSA/ACT binding phage (Clackson, T. et al., (1991) Nature. **352**: 624-628; Griffiths, A.D. et al., (1994) EMBO J. **13**: 3245-3260) or soluble sFv (Marks, J.D. et al., (1991) J. Mol. Biol. 222: 581-597). Immulon 4 Plates (Dynatech) were coated with 5-10µg/ml antigen. Phage binding was detected as with polyclonal phage. Binding of sFv was detected with the HRP-conjugated mouse mAB9E10 (Boehringer Mannheim) which recognizes the C-terminal Myc peptide tag (Munro, S. & Pelham, H.R.B., (1986) Cell. 46: 291-300). To assay for PSA binding, Costar 3590 high binding microtiter plates were coated with 2-5 µg/ml antigen. Clones were compared in phage ELISA binding to PSA/ACT, ACT, and to PSA.

To account for the possibilities of antigen malformation, in addition to coating immunoplates with PSA/ACT, ELISAs were done on both phage and soluble sFv using reacti-bind streptavadin-coated polystyrene microtiter plates (Pierce) and either bio-PSA/ACT or cocktails of the bio-Fabs to capture the complex. Plates were coated overnight with a total of 10µg/ml (5µg/ml of each derivative) of either Bio-PSA/ACT or bio-ACT or Bio-PSA. Plates were also coated with a cocktail of bio-Fabs at 5µg/ml. Unmodified PSA/ACT was then added at 1µg/ml and incubation continued for at least 90 minutes. Phage or soluble sFv were detected as described above.

Clones were screened by PCR (Gussow, D. & Clackson. (1989) T. Nucleic Acids Research. 17: 4000) and fingerprinted with restriction enzyme BSTN1 (restriction pattern nanalyzed) to identify different clones (Clackson, T. et al., (1991) Nature. 352: 624-628). Inserts were amplified using primers LMB3 and FdSeq as described in Marks, J.D. et al., (1991) J. Mol. Biol. 222: 581-597. Examples of clones with different restriction patterns were selected and sequenced. Sequencing templates were prepared by Qiagen plasmid midi kit and sequenced using the ABI Prism Dye Terminator Cycle Sequencing Ready Reaction Kit with amplitaq FS (ABI/Perkin Elmer).

### 1.5 Phage Preparations

Larger scale phage preps of selected clones were performed essentially as the library rescue. Twelve ml of 2XTY/ 100µg/ml Amp/ 1% glucose were inoculated with individual clones and the culture grown at 37°C to an OD600=0.5-0.7 and helper phage were added. After 30 minutes at 37°C without shaking, infected cells were pelleted (3000g for 10 minutes) and resuspended in 50ml 2XTY containing 100µg/ml Amp and 25µg/ml Kanamycin. Phage were propagated overnight at 30°C. 40 ml of overnight culture was centrifuged at 3300 g for 30 minutes, 1/5 volume of PEG/NaCl (20% PEG 6000, 2.5M NaCl) was added to the supernatant, mixed and left on ice for at least 1 hr. Phage were then pelleted at 3,300 g for 30 minutes, supernatant was removed and the pellet was resuspended in 2 ml PBS. A 10 minute top speed spin in an eppendorf micro centrifuge removed most of the remaining bacterial debris.

### 1.6 sFv Expression and Purification

Plasmid DNA was prepared (Qiagen kit) from selected clones and transformed into the non-supressor strain HB2151. Individual clones in either TG-1 or HB2151 were used to inoculate 500 ml cultures in 2XTY/Amp/0.1% Glucose. At mid-log phase, soluble sFv expression was induced (sFv under the control of the lacZ promoter) with 1mM IPTG overnight at 30°C. The periplasmic fraction which should contain the soluble sFv following IPTG induction was prepared from 5 ml of cells by cold osmotic shock according to the method of Sawyer, J.R. & Blattner, F.R., (1991) Protein Engineering **4**: 947-953, with the lysozyme omitted. HIS-tagged sFv were purified on NTA-Agarose (Qiagen).

### RESULTS

Polyclonal phage from both screening strategies demonstrated increased ELISA binding to PSA/ACT with each round of selection, as would be expected. Binding to ACT alone appeared to give a lower ELISA signal after subtraction. No PSA binding was detected.

Phage from individual clones were used in three types of ELISAs : PSA/ACT immobilized on immunoplates, biotinylated PSA/ACT on streptavin coated plates and PSA/ACT presented by biotinylated Fabs as described in methods. In general many clones performed well. Candidates from the soluble phase assayed well on immunoplates while immunotube selected phage bound streptavidin-biotin presented material or bio-Fab presented antigen as well.

Promising candidates were then used in comparative ELISAs in which phage were assayed for binding to PSA/ACT, ACT and PSA. Clones which demonstrated at least 4 fold greater binding to PSA/ACT over ACT were carried along for further analysis.

When no PSA binding was detected, even with the anti-PSA biotinylated Fabs, the immobilization of PSA was investigated. Only the Costar plates seem to be appropriate for this assay. Because of a concern with the immobilization of PSA on plastic, two rounds of soluble subtraction of PSA binders from the immunotube selected repertoire were further performed.

Individual clones were further analyzed. ELISA signal strength and complex-specificity (PSA/ACT versus ACT) was compared with BSTN1 fingerprints (restriction patterns) of amplified inserts to narrow down the field of candidates. Four candidates from the immunotube selection (IT), (representing three fingerprint patterns) and four from the soluble phase selection (BIO) (two fingerprint patterns) were initially characterized. Following sequence analysis, 5 clones (see Table 1) were further characterized. All of the clones with the same fingerprints had the same sequences. Sequence data also demonstrated that each of the selections had yielded V regions from a variety of human subclasses (Table 1).

**TABLE 1**

| Variable Region Subclass Usage of Selected Clones | | | |
|---|---|---|---|
| **SELECTION** | **CLONE** | **HUMAN SUBGROUP** | |
| | | **VH** | **VL** |
| IMMUNOTUBE | ITA3 | III | λI |
| | ITA2 | I | κI |
| | ITA7 | I | κI |
| SOLUBLE | BIOA8 | I | κII |
| | BIOC7 | II | λI |
| The complete sequence of each selected single chain antibody was compared with Human variable region heavy chain sequences and light chain sequences listed in Kabat ("Sequences of Proteins of Immunological Interest") to determine how many different subclasses were represented in the selected pool of phage displayed antibody fragments. | | | |

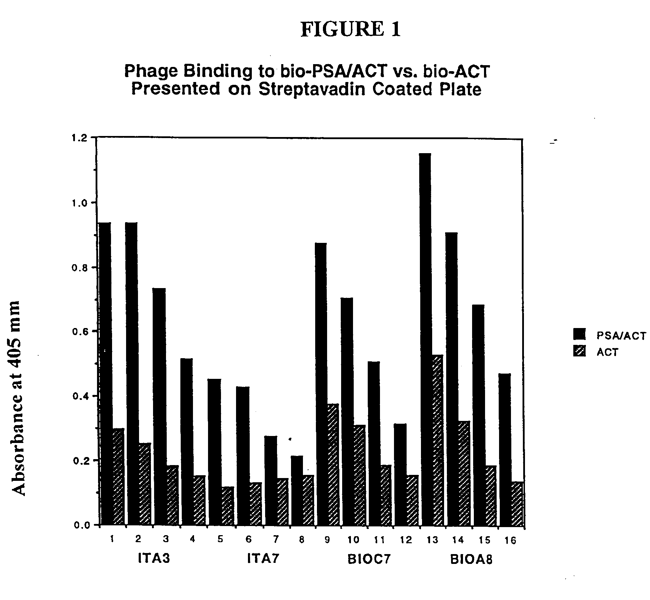

Larger scale phage preps (50 ml) were done on each of the 5 clones. These were used in dilution studies to confirm the binding profiles of each of the candidates. Figure 1 is a bar graph depicting phage binding to bio-PSA/ACT versus bio-ACT presented on streptavidin coated plate. Biotinylated PSA/ACT and biotinylated ACT at 5 µg/ml were each immobilized on separate halves of a reacti-bind streptavidin coated polystyrene microtiter plate (Pierce). Each phage preparation (6x10¹⁰-1x10¹¹ phage/ml) underwent threefold serial dilution and was added to each half of the plate. Phage were allowed to bind for at least 90 minutes. The plate was washed and phage were detected with anti-m13-HRP conjugate and ABTS substrate. Signal (absorbance) was read at 405 nm. For each phage prep, a total of 4 dilutions (undiluted and 3, threefold serial dilutions) are shown in Figure 1. (In each of the assays shown in Figures 1-4, binding signal (absorbance) was read at 405 nm and a total of 4 dilutions (undiluted and 3, threefold serial dilutions) are shown). The results in Figure 1 show that PSA/ACT binding was significantly higher than binding to ACT.

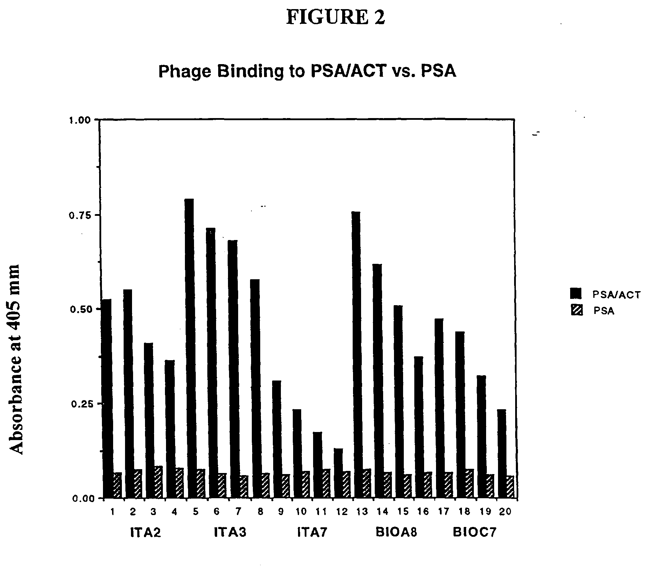

In addition, phage were assayed for binding to free PSA on a Costar High Binding E.I.A. Plate (Fig 2). Figure 2 shows phage binding to PSA/ACT versus PSA. A Costar high binding E.I.A. plate was coated overnight with PSA/ACT on one side, and free PSA on the other side of the plate, each antigen at 2.5 µg/ml. Each phage preparation ( 6x10¹⁰-1x10¹¹ phage/ml) underwent threefold serial dilution and was added to each side of the plate. Phage were allowed to bind for at least 90 minutes. The plate was washed and phage were detected with anti-m13-HRP conjugate (1:5000) and ABTS substrate. As a control for PSA immobilization, a cocktail of 2 biotinylated anti-PSA Fabs (Biotin.MAB〈PSA〉 M 10 -Fab and Biotin.MAB〈PSA〉 PR 1 -Fab) was used in serial dilution starting from 1:150. Bio- Fab binding was detected with a streptavadin-HRP conjugate (Pierce) (1:4000).

To confirm the immobilization of the PSA, a cocktail of 2 biotinylated anti-PSA Fabs (Biotin.MAB〈PSA〉M 10-Fab and Biotin.MAB〈PSA〉 PR 1-Fab) which bind both PSA and PSA/ACT was used as a control. Fab binding was detected using a streptavadin-HRP conjugate. Both Fabs are known to bind the complex with higher affinity but the data clearly shows that PSA binding was detectable with the Fab cocktail and that our selected clones demonstrated low cross-reactivity.

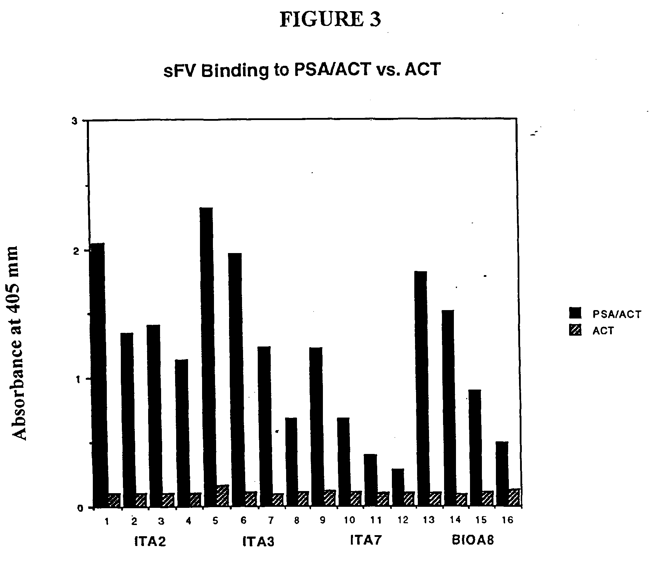

Following IPTG induction of sFv expression, the periplasmic fraction was prepared from each candidate clone and used in comparative ELISA. Figure 3 is a bar graph depicting sFv binding to PSA/ACT versus ACT. 5µg/ml of each antigen was immobilized on 1/2 of an Immulon 4 Flat-Bottom Immunoassay plate. sFv preps at 60-140µg/ml underwent threefold serial dilution and were added to each half of the plate. sFv binding was detected with the anti-Cmyc 9E10-HRP conjugate and ABTS substrate. The data in Figure 3 demonstrated that while complex-specific binding decreases with dilution of the extracts as would be expected, ACT binding appears to represent a fairly consistent background level. In addition it can be seen that candidates isolated from the immunotube selection (ITA2 & ITA3) recognize biotinylated antigen presented on a streptavidin plate, while candidates from the other selections bind to immobilized PSA/ACT.

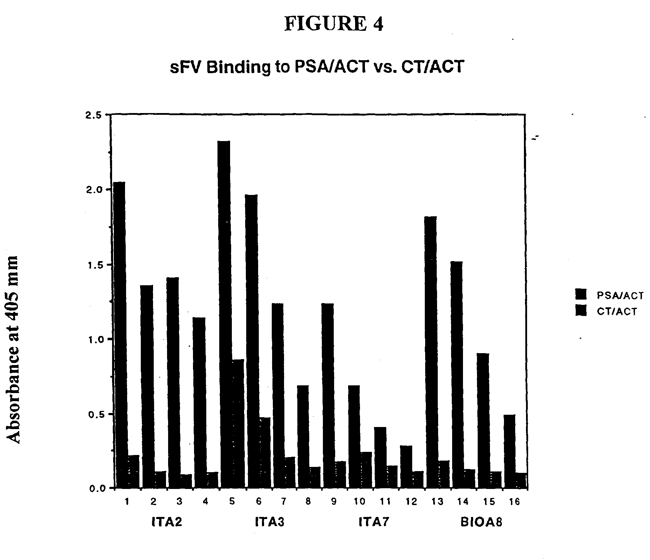

Having selected complex-specific phage displayed antibodies which recognize epitopes not present on the free subunit (PSA or ACT), we then assayed each of the antibodies for their ability to discriminate PSA/ACT from ACT complexed to other serine proteases, specifically chymotrypsin. Chymotrypsin-ACT (CT/ACT) was provided by Boehringer Mannheim, Germany along with MAb〈PSA-ACT〉M-4.6.374-IGG (as control) which cross-reacts with CT/ACT. Single chain expression was induced in selected clones. Following purification on NTA-resin, the sFvs were also used in competitive ELISAs. Dilutions of sFv preps (60-140µg/ml) were compared for binding to PSA/ACT and CT/ACT on immulon plates (Figure 4). In this assay (Figure 4), 5µg/ml of each antigen (PSA/ACT or CT/ACT (chymotrypsin-antichymotrypsin)) was immobilized on 1/2 of an Immulon 4 Flat-Bottom Immunoassay plate. sFv preps at 60-140µg/ml underwent threefold serial dilution and were added to each section of the plate. sFv binding was detected with the anti-c-myc 9E10-HRP conjugate and ABTS substrate. The clones demonstrated high specific binding to PSA/ACT. The low level of cross-reactivity was similar to that shown by the control antibody.

Lastly, the isolated antibody fragments are able to bind PSA/ACT at pH 7.4.

The results attest to the successful use of phage display antibody technology for the isolation of antibodies directed against the PSA/ACT complex. The strategies used to deplete the selected library of phages binding only to PSA or to ACT alone were effective. Antibody fragments displayed on phage or expressed as soluble sFv in the periplasm demonstrated the same complex-specificity. Adherence to plastic does not seem to alter the PSA/ACT complex in a manner that was detrimental to the assays. All of the selected sFv candidates bind PSA/ACT immobilized on polystyrene, biotinylated PSA/ACT presented on streptavidin, or unmodified PSA/ACT presented by non complex specific Fabs which should obviously be binding at non-overlapping epitopes.

### Example 2

### Subcloning and Mutagenesis

In general single chain antibodies vary in their levels of stability and tendencies to aggregate. The variable regions from the selected sFvs are subcloned into vectors for the expression of larger immunoglobulin (IG) derivatives. Vectors are available which enable the conversion of the presently isolated sFv clones into Fab fragments or even intact immunoglobulins if such are required for an assay format. Fusion proteins of the immunoglobulin derivatives are readily prepared using procedures known in the art.

Sequencing revealed that ITA7 clone had internal amber mutations. To maximize expression of these antibody fragments these amber codons are repaired by directed mutagenesis.

### Example 3

### Characterization of Binding

Epitope mapping is done to compare the anti-PSA/ACT antibodies of the present invention to other previously described antibodies which show complex binding, and to see how many non-overlapping complex-specific epitopes is identified by our panel of clones. Epitope mapping is done by competitive immunoassay or as described for example, in Immunoassay (E. P. Diamandis & T.K. Christopoulos, eds., Academic Press, Inc., 1996), pages 231-232 and 549.

Equilibrium and kinetic off-rates have been measured successfully with phage (Hawkins, R.E., et al., (1992) J. Mol. Biol. **226**: 889-896). Affinity studies are carried out by surface plasmon resonance (BIAcore) on purified monomers of each sFv. Equilibrium dialysis can also be employed for a Scatchard analysis. These methods have been described.

### Example 4

### Functional Assay

The present PSA/ACT complex antibodies are assayed for ability to detect PSA/ACT complexes in patient samples, such as by ELISA. The various potential configurations for the immunoassays are described in the art. The principle of the Elecsys® system immunoassay is described above. In one format of a diagnostic immunoassay using the Elecsys® system, a PSA-ACT antibody is immobilized on the magnetic particles. A patient sample containing the PSA-ACT to be measured is added to the antibody-coated magnetic particles. In addition, a labeled PSA-ACT complex is added to compete with the patient PSA-ACT for the antibody for the antibody binding site. After incubation, the sample is moved to the measuring cell washed and the amount of PSA-ACT complex measured as described above. The concentration/amount of bound complex is inversely related to the measured light output.

Results are compared to commercial assays now on the market.

## Claims

1. A method for obtaining an antibody specific for an antigen complex comprising a first subunit and a second subunit, comprising the steps of:
a) providing a library of antibodies displayed by a recombinant replicative vector particle;
b) selecting vector particles from the library that display antibody specific for the antigen complex alone, by contacting the library with the complex and recovering the vector particles that bind the complex;
c) removing vector particles from the library that display antibody specific for the first subunit alone, by contacting the library with the first subunit and recovering the unbound particles;
d) removing vector particles from the library that display antibody specific for the second subunit alone, by contacting the library with the second subunit and recovering the unbound particles;
e) clonally replicating particles that are present in the library after processing according to steps b), c), and d); and
f) selecting clones of replicating particles obtained following step e) that display antibody that binds the complex but not the first or second subunit alone.

2. The method of claim 1, wherein the library of antibodies is a naïve library.

3. The method of claim 2, wherein the library of antibodies comprises an immunoglobulin V region repertoire of at least 10⁸ members.

4. The method of claim 1, wherein the antibodies displayed by the library are single chain variable regions.

5. The method of claim 1, further comprising enriching the library that remains after step d), for vector particles that display antibody specific for the complex alone by contacting the vector particles with the complex and recovering the vector particles that bind the complex.

6. The method of claim 4, comprising the additional step of expressing the heavy and light chain variable regions displayed by the particle selected in step f) on separate polypeptide chains that reassemble to form a variable region that binds the complex.

7. The method of claim 1 wherein the antigen complex is prepared and contacted with the library under conditions that favor the stability of the complex in vitro.

8. The method of claim 1, wherein the recombinant replicative vector particle is a filamentous phage.

9. The method of claim 1, wherein the first subunit is prostate-specific antigen and the second subunit is anti-chymotrypsin.

10. The method of claim 9, wherein the library of antibodies is a naïve human antibody library.

11. The method of claim 9 wherein the antigen complex was prepared and contacted with the library at 4°C and pH 6.0.

12. An antibody prepared according to the method of claim 1, wherein the antibody binds PSA-ACT.

13. An antibody recombinantly produced to express the heavy and light chain variable region genes isolated from a particle selected in step f) of claim 1, wherein the antibody binds PSA-ACT.

14. The antibody of claim 13 selected from the group of antibodies consisting of Fab fragment, intact antibody, fusion antibody, and chimeric antibody.

15. An antibody that binds a complex formed between prostate specific antigen (PSA) and anti-chymotrypsin (ACT) with an affinity at least 10 fold higher than the affinity for either PSA or ACT alone.

16. An antibody according to claim 15 that binds the complex with an affinity at least 10³ fold higher than the affinity for either PSA or ACT alone.

17. An antibody according to claim 15 that binds the complex with an affinity at least 10 fold higher than the affinity for a complex between other serine proteases and ACT.

18. An antibody according to claim 15 that binds the complex with an affinity at least 10 fold higher than the affinity for a complex between chymotrypsin and ACT.

19. An antibody according to claim 15, which is a human antibody.

20. An antibody according to claim 15, which is a single chain variable region.

21. An antibody according to claim 15, in which the heavy and light chain variable regions are present on separate polypeptide chains.

22. An antibody according to claim 15, having the heavy and light chain variable regions of an antibody selected from the group of antibodies consisting of ITA2, ITA3, ITA7, BIOA8, and BIOC7.

23. An antibody according to claim 15 that competes with an antibody according to claim 15 for binding to the PSA-ACT complex.

24. An antibody according to claim 15, having the heavy and light chain complementarity determing regions (CDRs) of an antibody selected from the group of antibodies consisting of ITA2, ITA3, ITA7, BIOA8, and BIOC7.

25. An antibody according to claim 15, wherein the selected antibody is ITA2 having the amino acid sequence of SEQ ID. NO. 2.

26. An antibody according to claim 15, wherein the selected antibody is ITA3 having the amino acid sequence of SEQ ID. NO. 5.

27. An antibody according to claim 15, wherein the selected antibody is ITA7 having the amino acid sequence of SEQ ID. NO. 8.

28. An antibody according to claim 15, wherein the selected antibody is BIOA8 having the amino acid sequence of SEQ ID. NO. 11.

29. An antibody according to claim 15, wherein the selected antibody is BIOC7 having the amino acid sequence of SEQ ID. NO. 14.

30. A method of using an antibody according to claim 15 for determining the presence or amount of complex between PSA and ACT in a sample, comprising preparing a reaction mixture comprising the antibody and the sample under conditions that permit a PSA-ACT complex to bind the antibody, and determining the presence or amount of any PSA or ACT bound to the antibody in the reaction mixture.

31. A method for distinguishing between a benign and malignant prostate condition in a patient, comprising measuring the amount of complex between PSA and ACT in a sample obtained from the patient, according to the method of claim 30, and correlating the amount of the complex with non-malignancy of the condition.

32. A method according to claim 31, wherein the sample is a serum sample.

33. A method for distinguishing between a benign and malignant prostate condition in a patient, comprising determining the amount of total PSA in a sample obtained from the patient, measuring the amount of complex between PSA and ACT in the sample according to the method of claim 30, and correlating the ratio of the complex to the total PSA with non-malignancy of the condition.
